(19) Europäisches Patentamt
European Patent Office
Office européen des brevets

(11) EP 4 308 929 B1

(12) EUROPEAN PATENT SPECIFICATION

(45) Date of publication and mention of the grant of the patent:
**12.11.2025 Bulletin 2025/46**

(21) Application number: **22717454.7**

(22) Date of filing: **21.03.2022**

(51) International Patent Classification (IPC):
***G01N 33/542*** *(2006.01)* ***C12Q 1/37*** *(2006.01)*
***C12Q 1/04*** *(2006.01)* ***G01N 33/569*** *(2006.01)*

(52) Cooperative Patent Classification (CPC):
**G01N 33/542; C12Q 1/04; C12Q 1/37; G01N 33/56911**

(86) International application number:
**PCT/NL2022/050149**

(87) International publication number:
**WO 2022/197186 (22.09.2022 Gazette 2022/38)**

(54) **METHOD FOR THE IN VITRO DIAGNOSIS OF INFECTION**

VERFAHREN ZUR IN-VITRO-DIAGNOSE VON INFEKTIONEN

PROCÉDÉ DE DIAGNOSTIC IN VITRO D’UNE INFECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2021 NL 2027785**

(43) Date of publication of application:
**24.01.2024 Bulletin 2024/04**

(73) Proprietor: **Original G B.V.**
**9712 BP Groningen (NL)**

(72) Inventors:
- **GAZENDAM, Joost Alexander Christiaan**
**9712AN Groningen (NL)**
- **BURTON, Matthew Francis**
**9712AN Groningen (NL)**

(74) Representative: **Wittop Koning, Tom Hugo**
**Biopatents IP Consultancy**
**Dagpauwooglaan 1**
**5691NW Son en Breugel (NL)**

(56) References cited:
**WO-A1-2010/035001**
**WO-A1-2018/224561**
**WO-A2-2010/002976**
**WO-A2-2016/076707**
**US-A1- 2007 275 423**
**US-A1- 2011 213 121**
**US-A1- 2011 263 975**

- **RODRIGUES ET AL: "Evaluation of trehalose and sucrose as cryoprotectants for hematopoietic stem cells of umbilical cord blood", CRYOBIOLOGY, ACADEMIC PRESS INC, US, vol. 56, no. 2, 26 January 2008 (2008-01-26), pages 144 - 151, XP022559274, ISSN: 0011-2240**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 308 929 B1

## Description

### Field of the invention

The present invention is set out in the appended set of claims and relates to a method for in vitro diagnosing infection in bodily fluid samples, a lyophilized bead comprising a reagent and carbohydrate, a system for detecting the presence of infection in a bodily fluid and a kit for detecting the presence of infection in a bodily fluid.

### Background art

There is an increasing clinical need for improved diagnostic methods for detecting infection in bodily fluid samples. With the rise of antibiotic resistance, it is becoming increasingly important to diagnose infection at an early stage before biofilms develop. A biofilm is a layer comprising any syntrophic consortium of microorganisms in which cells stick to each other and often also to a surface of a medical device. Such a layer is difficult to treat with antibiotics. Enabling early diagnosis of an infection can slow biofilm formation / improve the efficacy of antibiotic treatment.

A particular need for rapid infection diagnosis is in the area of implanted medical devices. For example, with infections associated with joint replacement. Approximately 1.5 million total hip replacement (total hip arthroplasty - THA) operations are carried out world-wide annually. This is likely to increase to approximately 3 million worldwide per annum within the next decade. In addition, other types of implants and joint replacements, such as knee, shoulder, foot, ankle, hand, wrist, elbow, cranio-maxillofacial and dental, are also being used in increasing amounts. Globally, the infection rate for THA is 1-16 %, typically in the USA and Europe the infection rate is 3-12%, although in more elderly patient groups the infection rate for THA rises to 24%. Early, post-operative diagnosis of infection of implanted medical devices is desired in order to avoid difficult to treat infections and revision surgery.

In addition, known tests e.g. Synovaure® (Zimmer Biomet) aimed at identifying the presence of an infection such as periprosthetic joint infection measure biomarkers of the host (human) immune system response to infection, and thus do not directly detect infections (bacteria). A downside of indirect testing directed to host (human) immune system response is that in the acute phase (first couple of weeks post operation) it is not possible to differentiate from host (human) immune system inflammatory response and the host (human) immune system infection response.

Likewise, medical devices that are implanted in the spine are also prone to infection. Postoperative spine infection can be a devastating complication after spine surgery in both the short term and long term. Infection places a patient at a high risk for pseudoarthrosis, chronic pain, return to operating room, adverse neurological sequelae, worsened long-term outcomes, and even death. Depending on the type of spine surgery being performed, the incidence of infection is highly variable, with reported ranges listed up to 18%. Posterior cervical surgery has a higher rate of infection than posterior lumbar surgery and anterior spinal surgery.

Another clinical group in need of rapid infection diagnosis are patients undergoing peritoneal dialysis. Peritoneal dialysis (PD) has advantageous over haemodialysis, for example provision of peritoneal dialysis is generally less expensive and has fewer negative side effects (such as nausea, vomiting, cramping, and weight gain). However, the disadvantage of PD is peritonitis. Peritonitis is a common complication of peritoneal dialysis. It's associated with significant morbidity, catheter loss, transfer to haemodialysis, transient loss of ultrafiltration, possible permanent membrane damage, and occasionally death. Peritonitis may be directly related to peritoneal dialysis or secondary to a non-dialysis-related intra-abdominal or systemic process. Most cases are peritoneal dialysis related. Peritoneal dialysis-related peritonitis is either due to contamination with pathogenic skin bacteria during exchanges (i.e., touch contamination), or to an exit-site or tunnel infection. Secondary peritonitis is caused by underlying pathology of the gastrointestinal tract and occasionally (albeit rarely) due to haematogenous spread (i.e., following dental procedures). Causes of secondary peritonitis include cholecystitis, appendicitis, ruptured diverticulum, treatment of severe constipation, bowel perforation, bowel ischemia, and incarcerated hernia. Rapid diagnosis of peritonitis is important in order begin antibiotic treatment as early as possible.

Currently, bodily fluid samples need to be subject to complex, time consuming analytical techniques or microbiological assays, which means that a rapid diagnosis cannot be carried out at the point of care. Use of lateral flow devices is hampered by sample inhomogeneity and contamination with blood in traumatic samples.

WO 2016/076707 discloses an object surface coating in which the cleavage of the first cleavage site simultaneously results in the release of the first non-quenching agent from the object surface coating so that emission of the first fluorescent agent can be detected. WO 2016/076707 teaches a heterogenous system for detecting infection in vivo. WO 2016/076707 does not relate to an in vitro diagnostic method.

WO 2018-224561 teaches a method for detecting food spoilage microbes the method comprising:

a) adding a first pH adjustment agent to a food sample to provide a food sample having a pH in the range of pH 1 to 5, separating any solid precipitate present in the pH adjusted food sample to provide a pH adjusted food sample and adding a second pH adjustment agent to the pH adjusted food sample to provide a food sample having a pH in the

range of pH 6.5-9 to be used in step b),
b) contacting a food sample with a peptide substrate. WO 2018-224561 defines food spoilage as "an unsatisfactory change in the sensory characteristics of a food stuff". WO 2018-224561 relates to a different field than in vitro diagnostic methods of bodily fluids.

WO 2018-085895 discloses chemiluminescent BRET based sensors for detection of proteases in UHT and raw milk; the Examples show exclusively UHT and raw milk. The proteases detected are extracted from the food spoilage organism *Pseudomonas fluorescens.* WO 2018-085895 relates to the field of food spoilage, in particular a biosensor for detection of *Pseudomonas fluorescens* in milk. Moreover, WO 2018-085895 relates to a different field than in vitro diagnostic methods of bodily fluids and teaches detection using the RLuc2/Clz400a / GFP2 system at wavelengths 410/ 515 nm.

US 2007/275423 discloses a synthetic enzyme substrate, human alpha-1 protease inhibitor, that can be used in a test system to identify multiple microorganism species, such as bacteria, that commonly infect wounds.

There is a need for a rapid, in vitro diagnostic method that identifies infection in bodily fluid samples and that can be carried out preferably be carried out at the point of care. In addition, there is a need for a rapid, in vitro diagnostic method that can be carried out in without surface effects.

There is also a rapid, in vitro diagnostic method that can be carried out in samples contaminated with blood.

In particular, there is also a need for a rapid, diagnostic method that can identify the presence of periprosthetic joint infection, peritoneal dialysis-related peritonitis and/or cerebrospinal associated infection in a sample of synovial fluid, peritoneal fluid or cerebrospinal fluid sample in the acute phase following surgery.

**Summary of the invention**

The present invention provides a method for in vitro diagnosing infection in bodily fluid samples using a reagent having the general formula [a]-[b]-[c] (I), wherein:

[a] is a fluorescent agent having an emission wavelength of 650-900 nm,
[c] is a non-fluorescent agent having an absorption wavelength of 650-900 nm, for quenching said emission of said fluorescent agent,
[b] is a peptide comprising a cleavage site, the cleavage site being specific for a bacterial biomarker, wherein the bacterial biomarker is a bacterial membrane bound protease, bacterial membrane bound transpeptidase, intracellular bacterial protease or extracellular bacterial protease, the reagent being provided in the form of a lyophilized bead comprising a carbohydrate, chosen from the group, consisting of monosaccharide, disaccharide, polysaccharide and combinations thereof, the monosaccharide having the general formula $(CH_2O)_n$

wherein the method comprises the steps of:

i) contacting a sample of bodily fluid, preferably human bodily fluid, with the reagent in a sample receptacle, and
ii) monitoring the fluorescence emission in the range of 650-900 nm from the sample in step i), wherein an increase in fluorescence emission in the range of 650-900 nm is indicative for the presence of infection in the bodily fluid sample.

According to the method is defined herein, rapid in vitro diagnosis of infection in bodily fluid samples is made possible by analysing the fluorescence emission in the range of 650-900 nm from the reagent in the presence of a samples of bodily fluid. When the target bacterial biomarker is present in the bodily fluid, the bacterial biomarker recognises and cleaves the cleavage site in the reagent, increasing the distance between the fluorescent and non-fluorescent agent, and as a result a fluorescent signal is emitted, which can be detected by a suitable detector.

Surprisingly, it has been found that the present method can be carried out directly in a bodily fluid sample without the need to enrich the bacterial cell count by, for example, a bacterial enrichment step. As a result, the present method is able to detect bacterial infection rapidly, for example, within 30 minutes, preferably within 15 minutes, of contacting the reagent with sample.

A further advantage of the present method is that the method can be run directly in the sample, that is without a sample clean-up step, as the presence of components such as red blood cells which interfere with UV wavelength based detection methods, do not cause interference with the present method.

In a second aspect, there is provided a lyophilized bead as defined in any of the appended claims comprising a carbohydrate, chosen from the group, consisting of monosaccharide, disaccharide, polysaccharide and combinations thereof, the monosaccharide having the general formula $(CH_2O)_n$ and a reagent having the structure [a]-[b]-[c] (I) wherein:

[a] is a fluorescent agent having an emission wavelength of 650-900 nm

[c] is a non-fluorescent agent having an absorption wavelength of 650-900 nm, for quenching said emission of said fluorescent agent, [b] is a peptide comprising a cleavage site, the cleavage site being specific for a bacterial biomarker.

In a third aspect there is provided a kit for in vitro infection in bodily fluids, comprising:

a) a container comprising a lyophilized bead of as described above,
b) a set of instructions for carrying out the method as described herein.
c) optionally a container containing a diluent, the reagent and the diluent preferably being present in two compartments of a container, preferably wherein the compartments are separated by a pressure activatable membrane.

In a fourth aspect, there is provided a system, comprising:

a) a container comprising a lyophilized bead as described above,
b) a device adapted to receive the container and monitor the fluorescence signal emitted from the reagent in the presence of a sample of bodily fluid with the reagent, wherein the system is adapted to carry out a method as described herein.

**Description of embodiments**

The term "subject" as used herein means an animal or human individual who is at risk of or suspected of having an infection. The terms "patient," "subject," "individual," and the like are used interchangeably herein, and refer to any animal or human amenable to the methods described herein. In certain non-limiting embodiments, the patient, subject or individual is a human.

The term "bodily fluid sample" as used herein means a biological material isolated from a subject in the fluid state.

The term "biomarker" as used herein means an enzyme, transpeptidase, peptidase or protease that is bound to, or excreted from a cell or microorganism, The biomarker is indicative of the status of a particular analyte or subject, for example the biomarker is indicative of the presence of a particular bacteria, or is indicative of a particular clinical situation, for example the clinical situation may be an infection, disease state or metabolic state.

The term "bacterial biomarker" as used herein means a transpeptidase, peptidase or protease that is bound to, or excreted from a bacterium or group of bacteria. The bacterial biomarker is indicative for the presence of infection in a bodily fluid, preferably periprosthetic joint infection, peritoneal dialysis-related peritonitis and/or cerebrospinal associated infection.

The terms "protease" and "peptidase" as used herein mean a protein present in bacteria, secreted by bacteria or present in the membrane of bacteria capable of cleaving an amino acid motif.

The term "transpeptidase" as used herein means a protein present in the membrane of bacteria capable of cleaving an amino acid motif of a first protein and covalently linking the cleaved amino acid motif to a second protein.

The term "peptide" as used herein means a reagent comprising at least 3 amino acids. Preferably, the peptide comprises no more than 20 amino acids. The amino acids used may be any amino acid, preferably chosen from the group of naturally occurring amino acids or from the group of synthetic amino acids, in particular derivatives of natural amino acids.

The term "cleavage site" as used herein means an amino acid motif that is cleaved by a specific compound whereby the cleavage site comprises one or more amide bonds. Preferably, the cleavage site contains 3 amino acids. Preferably, the cleavage site is cleaved by the action of a protease or transpeptidase.

"Sample" or "biological sample" as used herein means a biological material isolated from an individual. The biological sample may contain any biological material suitable for detecting the desired biomarkers, and may comprise cellular and/or non-cellular material obtained from the individual.

The term "monitoring", "measuring" "measurement," "detecting" or "detection," as used herein means assessing the presence, absence, quantity or amount (which can be an effective amount) of either a given substance within a clinical or subject-derived sample, including the derivation of qualitative or quantitative concentration levels of such substances, or otherwise evaluating the values or categorization of a subject's clinical parameters.

The term "infection" as used herein means a clinically relevant amount of bacteria which is indicative of a subject showing clinical signs of an infection.

The term "clinically relevant amount of bacteria" as used herein means a bacterial colony count of more than 0.1 CFU/mL, more than 1 CFU/mL, $1x10^1$ CFU/mL, more than $1x10^2$ CFU/mL, preferably more than $1x10^3$ CFU/mL, more preferably more than $1x10^4$ CFU/mL, even more preferably $1x10^5$ CFU/mL, yet more preferably $1x10^6$ CFU/mL, even yet more preferably $1x10^7$ CFU/mL, and even yet more preferably $1x10^8$ CFU/mL.

In a first aspect there is provided a method as defined in claim 1.

Preferably, the bodily fluid sample is selected from the group consisting of gingival fluid, amniotic fluid, urine,

serous fluid, synovial fluid, peritoneal dialysis fluid, semen, sebum and cerebrospinal fluid, preferably wherein the bodily fluid is selected from the group consisting of synovial fluid, peritoneal dialysis fluid and cerebrospinal fluid, even more preferably wherein the bodily fluid is selected from the group consisting of synovial fluid and peritoneal dialysis fluid.

Preferably, the sample is selected from the group consisting of cerebrospinal fluid, synovial fluid and peritoneal fluid.

Preferably, the bodily fluid sample is a serous fluid selected from the group consisting of pleural fluid, pericardial fluid, peritoneal fluid and combinations thereof.

In the context of the present invention, "serous fluid" means the fluid that is present in the the pleural, pericardial, and peritoneal cavities. These cavities are each lined by two membranes referred to as the serous membranes. One membrane lines the cavity wall (parietal membrane), and the other covers the organs within the cavity (visceral membrane). The fluid between the membranes is called serous fluid.

Samples of serous fluids are collected by needle aspiration from the respective cavities. These aspiration procedures are referred to as thoracentesis (pleural), pericardiocentesis (pericardial), and paracentesis (peritoneal).

It is known that serous fluids are formed as ultrafiltrates of plasma. Under normal conditions, oncotic pressure from serum proteins is the same in the capillaries on both sides of the membrane. Therefore, the hydrostatic pressure in the parietal and visceral capillaries causes fluid to enter between the membranes. The filtration of the plasma ultrafiltrate results in increased oncotic pressure in the capillaries that favours reabsorption of fluid back into the capillaries. This produces a continuous exchange of serous fluid and maintains the normal volume of fluid between the membranes. Examples of serous fluids are pleural fluid, pericardial fluid and peritoneal fluid.

Preferably, the method described herein relates to the in vitro diagnosis of infection in serous fluid.

Fluid found in the pleural cavity, located between the parietal pleural membrane lining the chest wall and the visceral pleural membrane covering the lungs, is referred to as pleural fluid. Preferably, the method described herein relates to the in vitro diagnosis of infection in pleural fluid, more preferably the method described herein relates to the in vitro diagnosis of bacterial pneumonia and/or tuberculosis.

Pericardial fluid is found between the pericardial serous membranes. The pericardial fluid may become infected. Preferably, the method described herein relates to the in vitro diagnosis of infection in pericardial fluid, more preferably the diagnosis of bacterial endocarditis and pericarditis.

Fluid between the peritoneal membranes is called ascites, and the fluid is commonly referred to as ascitic fluid or peritoneal fluid. Preferably, the method described herein relates to the in vitro diagnosis of infection in ascetic fluid (peritoneal fluid), more preferably the diagnosis of peritonitis.

In some circumstances, peritoneal fluid is provided by peritoneal washing or lavage by conventional transabdominal techniques readily familiar to the physician.

Within the context of the present application, "peritoneal dialysis fluid" means the fluid that has been infused into the peritoneal (abdominal) cavity as part of the technique known as continuous ambulatory peritoneal dialysis (CAPD) or continuous cycling peritoneal dialysis (CCPD; also known as automated peritoneal dialysis (APD) or another other similar technique. In these techniques, metabolic waste products and excess electrolytes and other materials are transferred from the body into a fluid infused into the peritoneal cavity, using the peritoneal membrane as a dialyzing membrane, over a period of time commonly referred to as the dwell time. The fluid for dialysis is introduced into the peritoneal cavity by a transabdominal connection. By virtue of the introduction of this artificial connection to the peritoneal cavity, these patients are exposed to an increased risk or peritoneal inflammation or peritoneal infection, commonly referred to as peritonitis.

Preferably, the method described herein relates to the in vitro diagnosis of infection in peritoneal dialysis fluid, more preferably the diagnosis of peritonitis associated with peritoneal dialysis.

The peritoneal dialysis fluid sample can be provided by any convenient means. If the patient is a CAPD patient, a sample of dialysate fluid can be taken as the waste (spent) fluid is removed from the peritoneal cavity. Alternatively, the peritoneal dialysis fluid sample can be provided by removing a sample via the sample port of the waste bag for collecting the peritoneal dialysis fluid after the stipulated dwell time has passed. Obtaining samples from such waste bags can be done using standard methods.

Within the context of the present application "Synovial fluid" means the biological fluid that is found in the synovial cavity of a joints between the cartilage and synovium of facing articulating surfaces. It is known that synovial fluid provides nourishment to the cartilage and also serves as a lubricant for the joints. The cells of the cartilage and synovium secrete fluid and the fluid lubricates and reduces friction between the articulating surfaces.

Preferably, synovial fluid is from a human or animal subject, more preferably from a human subject.

A typical human synovial fluid comprises approximately 85% water in addition to dissolved proteins, glucose, mineral ions, hormones, etc. The proteins, albumin and globulins, are typically present in synovial fluid and are believed to play an important role in the lubrication of the joint area. Other proteins are also found in human synovial fluid, including the glycoproteins such as alpha-1-acid glycoprotein (AGP), alpha-1-antitrypsin (A1AT) and lubricin. Another compound that is present in human synovial fluid is hyaluronic acid. Hyaluronic acid is also believed to play a role in lubrication. Human synovial fluid further includes other compounds, such as polysaccharides and phospholipids. The phospholipid, dipalmitoylphosphatidylcholine (DPPC), is also present in human synovial fluid. DPPC is generally regarded as surfactant and is also believed to play a role in the lubrication of the joint.

Bacterial contamination of synovial fluid can lead to sceptic arthritis. Preferably, the method described herein relates to the in vitro diagnosis of infection in synovial fluid, more preferably the diagnosis of sceptic arthritis.

In the case of bacterial contamination of synovial fluid in the presence of a joint replacement, this can lead to periprosthetic joint infection. Preferably, the method described herein relates to the in vitro diagnosis of infection in synovial fluid (peritoneal fluid), more preferably the diagnosis of peritonitis. periprosthetic joint infection (PJI).

Within the context of the present invention, "semen" refers to the section of male reproductive organs. An infection associated with semen is bacterial prostatis. Preferably, the method described herein relates to the in vitro diagnosis of infection in semen, more preferably the diagnosis of bacterial prostatis.

Amniotic fluid is the fluid that surrounds the foetus. An infection associated with amniotic fluid is intra-Amniotic infection. Preferably, the method described herein relates to the in vitro diagnosis of infection in amniotic fluid, more preferably the diagnosis of intra- Amniotic infection.

Urine is a liquid by-product of metabolism in humans and in many other animals. Urine flows from the kidneys through the ureters to the urinary bladder. Urination results in urine being excreted from the body through the urethra. Bacterial infection in urine is typically referred to as a urinary tract infection (UTI). Preferably, the method described herein relates to the in vitro diagnosis of infection in urine, more preferably the diagnosis of urinary tract infections.

Cerebrospinal fluid is the clear watery fluid which fills the space between the arachnoid membrane and the pia mater. Preferably, the method described herein relates to the in vitro diagnosis of infection in cerebrospinal fluid.

Preferably, the bodily fluid is gingival fluid. Preferably, the method disclosed herein relates to the in vitro diagnosis of gingivitis.

Preferably, there is provided a method according to claim 1 for in vitro diagnosing periprosthetic joint infection, peritoneal dialysis related peritonitis and/or cerebrospinal associated infection using a reagent having the general formula [a]-[b]-[c] (I)
wherein:

[a] is a fluorescent agent having an emission wavelength of 650-900 nm,
[c] is a non-fluorescent agent having an absorption wavelength of 650-900 nm, for quenching said emission of said fluorescent agent,
[b] is a peptide comprising a cleavage site, the cleavage site being specific for a bacterial biomarker, wherein the method comprises the steps of:

i) contacting a sample of synovial fluid, peritoneal fluid or cerebrospinal fluid with the reagent in a sample receptacle, and
ii) monitoring the fluorescence emission in the range of 650-900 nm from the sample in step i),

wherein an increase in fluorescence emission in the range of 650-900 nm is indicative for the presence of periprosthetic joint infection, peritoneal dialysis-related peritonitis and/or cerebrospinal associated infection.

Accordingly, there is provided a method according to claim 1 for in vitro diagnosing peritoneal dialysis-related peritonitis in a subject using a reagent having the general formula [a]-[b]-[c] (I)
wherein:

[a] is a fluorescent agent having an emission wavelength of 650-900 nm,
[c] is a non-fluorescent agent having an absorption wavelength of 650-900 nm, for quenching said emission of said fluorescent agent,
[b] is a peptide comprising a cleavage site, the cleavage site being specific for a bacterial biomarker,

wherein the method comprises the steps of:

i) contacting a sample of peritoneal fluid with the reagent a sample receptacle, and
ii) monitoring the fluorescence emission in the range of 650-900 nm from the sample in step i),

wherein an increase in fluorescence emission in the range of 650-900 nm is indicative for the presence of peritoneal dialysis-related peritonitis.

Yet another advantage of the method described herein is that peritoneal dialysis-related peritonitis is diagnosed by monitoring the fluorescence emission in the range of 650-900 nm from the sample of peritoneal dialysis fluid, enabling the method to be carried out directly on the collected peritoneal dialysis fluid after the necessary dwell time.

The advantage associated with the fluorescence emission being generated by cleavage of the cleavage site by a bacterial biomarker, is that the measured fluorescence emission is indicative of bacterial infection. In current clinical practice, the standard way for a patient or healthcare professional to check for bacterial presence is by observing whether the dialysis waste fluid is cloudy. However, the so-called cloudy bag test is prone to false positive results as a cloudy dialysis waste fluid may also be caused by elevated levels of either cellular or non-cellular constituents in the peritoneal dialysis fluid, such as elevated leucocyte and red blood cell counts. Consequently, the method described herein is able to distinguish between a bacterial infection (peritonitis) and inflammatory (non-infectious) disease states associated with peritoneal dialysis, as the fluorescence emission in the range of 650-900 nm is indicative of the presence of a bacterial biomarker.

Accordingly, there is provided a method according to claim 1 for in vitro diagnosing of periprosthetic joint infection in a subject using a reagent having the general formula [a]-[b]-[c] (I) wherein:

[a] is a fluorescent agent having an emission wavelength of 650-900 nm,
[c] is a non-fluorescent agent having an absorption wavelength of 650-900 nm, for quenching said emission of said fluorescent agent,
[b] is a peptide comprising a cleavage site, the cleavage site being specific for a bacterial biomarker,

wherein the method comprises the steps of:

i) contacting a sample of synovial fluid a sample receptacle, and
ii) monitoring the fluorescence emission in the range of 650-900 nm from the sample in step i),

wherein an increase in fluorescence emission in the range of 650-900 nm is indicative for the presence of periprosthetic joint infection.

Yet another advantage of the method described herein is that the periprosthetic joint infection can be diagnosed by monitoring the fluorescence emission in the range of 650-900 nm from the sample of synovial fluid, enabling the method to be carried out directly on the collected synovial fluid.

The advantage associated with the fluorescence emission being generated by cleavage of the cleavage site by a bacterial biomarker, is that the measured fluorescence emission is indicative of bacterial infection. State of the art methods to detect PJI typically detect elevated inflammatory biomarkers associated with bacterial infection. However, such methods based on inflammatory biomarkers cannot be used in the period immediately after a traumatic event or operation as the inflammatory biomarkers are elevated in this period. Consequently, methods based on inflammatory biomarkers are prone to false positive results. The method described herein is able to distinguish between a bacterial infection (PJI) and an inflammatory to trauma or an operation (e.g. joint replacement), as the fluorescence emission in the range of 650-900 nm is indicative of the presence of a bacterial biomarker. Consequently, the method described herein can be carried out within 8 weeks, preferably 6 weeks, preferably 4 weeks, preferably 2 weeks, preferably 1 week after a trauma or operation.

Accordingly, there is provided a method according to claim 1 for in vitro diagnosing cerebrospinal associated infection in a subject using a reagent having the general formula [a]-[b]-[c] (I)wherein:

[a] is a fluorescent agent having an emission wavelength of 650-900 nm,
[c] is a non-fluorescent agent having an absorption wavelength of 650-900 nm, for quenching said emission of said fluorescent agent,
[b] is a peptide comprising a cleavage site, the cleavage site being specific for a bacterial biomarker,

wherein the method comprises the steps of:

i) contacting a sample of cerebrospinal fluid a sample receptacle, and
ii) monitoring the fluorescence emission in the range of 650-900 nm from the sample in step i),

wherein an increase in fluorescence emission in the range of 650-900 nm is indicative for the presence of cerebrospinal associated infection.

Yet another advantage of the method described herein is that the infection in CSF can be diagnosed by monitoring the fluorescence emission in the range of 650-900 nm from the sample of synovial fluid, enabling the method to be carried out directly on the collected CSF.\

The advantage associated with the fluorescence emission being generated by cleavage of the cleavage site by a bacterial biomarker, is that the measured fluorescence emission is indicative of bacterial infection. State of the art methods to detect infection in CSF typically detect elevated inflammatory biomarkers associated with bacterial infection. However, such methods based on inflammatory biomarkers cannot be used in the period immediately after a traumatic event or

operation as the inflammatory biomarkers are elevated in this period. Consequently, methods based on inflammatory biomarkers are prone to false positive results. The method described herein is able to distinguish between a bacterial infection and an inflammatory to trauma or an operation (e.g. placement of spinal implant), as the fluorescence emission in the range of 650-900 nm is indicative of the presence of a bacterial biomarker. Consequently, the method described herein can be carried out within 8 weeks, preferably 6 weeks, preferably 4 weeks, preferably 2 weeks, preferably 1 week after a trauma or operation.

Yet another advantage of the method described herein is that the periprosthetic joint infection, by monitoring the fluorescence emission in the range of 650-900 nm from the sample of synovial fluid, enabling the method to be carried out directly on the collected synovial fluid.

Preferably, the bodily fluid sample comprises at least 70 wt.% water, more preferably at least 85% water, based on dry weight of the sample. Preferably, the bodily fluid sample comprises at least 70 wt.% and protein. Preferably, the bodily fluid comprises least 70 wt.% and protein, wherein if whey and casein proteins are present, these are present in a ratio in the range of 90:10 to 50: 50. Preferably, the whey:casein ratio is at least 25:75, preferably at least 30:70, more preferably at least 40:60.

Preferably, step ii) comprises monitoring the fluorescence emission in the range of 700-850 nm, more preferably in the range of 750-850 nm. Preferably, an increase in fluorescence emission in the range of 700-850 nm, more preferably in the range of 750-850 nm is indicative infection.

Preferably, an increase in fluorescence emission in the range of 700-850 nm, more preferably in the range of 750-850 nm is indicative for periprosthetic joint infection, peritoneal dialysis-related peritonitis and/or cerebrospinal associated infection.

The fluorescent agent having an emission wavelength of 650-900 nm is preferably a cyanine moiety (dye). Preferably, the non-fluorescent agent having an emission wavelength of 650-900 nm is a cyanine moiety (dye).

Preferably, the fluorescent agent having an emission wavelength of 650-900 nm comprises a cyanine moiety, preferably a sulfo-cyanine moiety and wherein the non-fluorescent agent having an absorption wavelength of 650-900 nm comprises a cyanine moiety, preferably a sulfo-cyanine moiety

Preferably, the fluorescent agent is a cyanine dye having an emission wavelength of 650-900 nm and the non-fluorescent agent is a cyanine dye having an absorption wavelength of 650-900 nm.

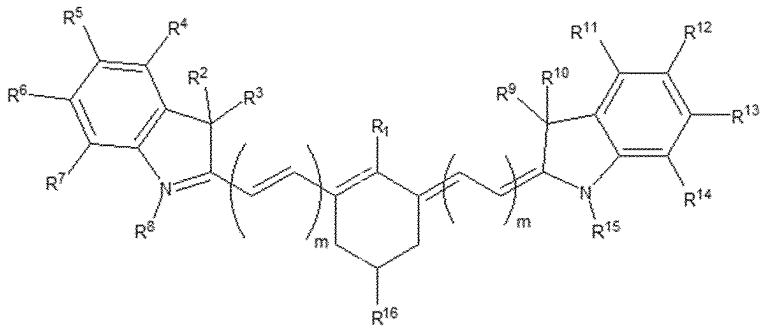

Formula I

In an embodiment, the first fluorescent agent is a cyanine dye having the general formula as shown in formula I, wherein $R^1$ is selected from the group consisting of H, halo, substituted phenyl ($R_{18}$-Ph-), wherein $R_{18}$ comprises a functional group (FG) that does not directly react with carboxyl, hydroxyl, amino or thiol groups, and

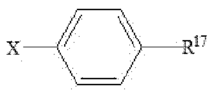

.

Preferably, $R_{18}$ is $(CH_2)_qR_{19}$FG moiety, wherein $R_{19}$ is a optionally substituted hydrocarbyl or amidyl moiety and FG is a functional group that does not directly react with carboxyl, hydroxyl, amino or thiol groups and q is an integer between 0 and 6, preferably between 1 and 5, more preferably between 2 and 4. $R_{19}$ is preferably selected from the group consisting of $(CH_2)_2CONH(CH_2)_2CONHFG$ wherein FG is a functionality selected from the group $-SO_3H$, triazine, cylcooctyne, azide, alkyne, tetrazine, alkene, alkene and tetrazole. Preferably, $R_{19}$FG is $CH_2)_2CONH(CH_2)_2CONH$-dibenzocyclooctyne.

X is selected from the group consisting of O, S, NH and N-hydrocarbyl; where $R^{17}$ is selected from the group consisting of carboxyl, amino and sulfanato; $R^2$, $R^3$, $R^9$,$R^{10}$ are each independently selected from the group consisting of H

and hydrocarbyl; $R^4$, $R^5$, $R^{11}$,$R^{12}$ are each independently selected from the group consisting of H, hydrocarbyl and sulfanato or together with the atoms to which they are bonded form an aromatic ring; $R^6$, $R^7$, $R^{13}$, $R^{14}$ are each independently selected from the group consisting of H and hydrocarbyl, $R^8$ and $R^{15}$ are each independently selected from the group consisting of hydrocarbyl, $(CH_2)$qFG or $(CH_2)_P$LN wherein at least one of $R^8$ and $R^{15}$ is $(CH_2)$qFG, wherein q is an integer from 1 to 20 and FG is a functional group that does not directly react with carboxyl, hydroxyl, amino or thiol groups, wherein p is an integer from 1 to 20 and LN is a linker group that reacts with carboxyl, hydroxyl, amino or thiol groups; $R^{16}$ is H or hydrocarbyl. Preferably, the functional group comprises a functionality selected from the group $-SO_3H$, triazine, cylcooctyne, azide, alkyne, tetrazine, alkene, alkene and tetrazole. Preferably the linker is selected from the group consisting of mercapto, amino, haloalkyl, phosphoramidityl, N-hydroxy succinidyl ester, sulfo N-hydroxysuccinimidyl ester, isothiocyanato, iodoacetamidyl, maleimidyl and an activated carboxylic acid.

Preferably, the fluorescent agent is an agent wherein $R^1$ is

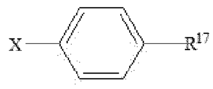

wherein X is O and $R^{17}$ is $SO_3Na$; $R^2$, $R^3$, $R^9$,$R^{10}$ are hydrocarbyl, preferably methyl; $R^4$ and $R^{11}$ are H and $R^5$ and $R^{12}$ are H or sulfanato; $R^6$, $R^7$, $R^{13}$, $R^{14}$ are H; R8 is $(CH_2)$qFG where q is 4 and FG is sulfanato;$R^{15}$ is $(CH_2)_P$LN where p is 5 and LN is carboxyl, $R^{16}$ is H.

Even more preferably, the fluorescent agent is an agent wherein $R^1$ is

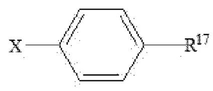

wherein X is O and $R^{17}$ is $SO_3Na$; $R^2$, $R^3$, $R^9$,$R^{10}$ are methyl; $R^4$ and $R^{11}$ are H and $R^5$ and $R^{12}$ are sulfanato; $R^6$, $R^7$, $R^{13}$, $R^{14}$ are H; R8 is $(CH_2)$qFG where q is 4 and FG is sulfanato; $R^{15}$ is $(CH_2)_P$LN where p is 5 and LN is carboxyl, $R^{16}$ is H.

Preferably, the fluorescent agent is an agent corresponding to formula II.

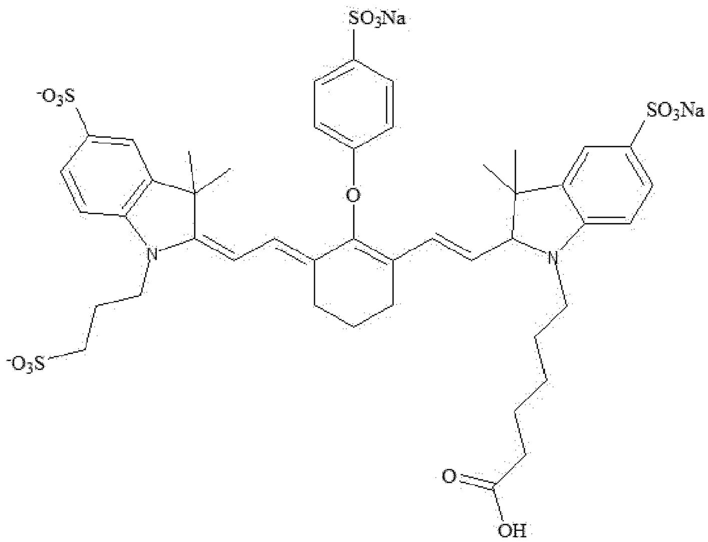

Formula II

Most preferably, the fluorescent agent is an agent wherein $R_1$ is $R_{18}$Ph, where $R_{18}$ is a N-[3-(11,12-didehydrodibenzo[b,f]azocin-5(6H)-yl)-3-oxopropyl]propanyl moiety, $R_{16}$ is H, $R_4$ and $R_{11}$ are H, $R_5$ and $R_{12}$ is $SO_3H$, $R_{12}$ is $(CH_2)_3SO_3H$, $R_6$, $R_7$, $R_{13}$, $R_{14}$ are H as shown in Formula V.

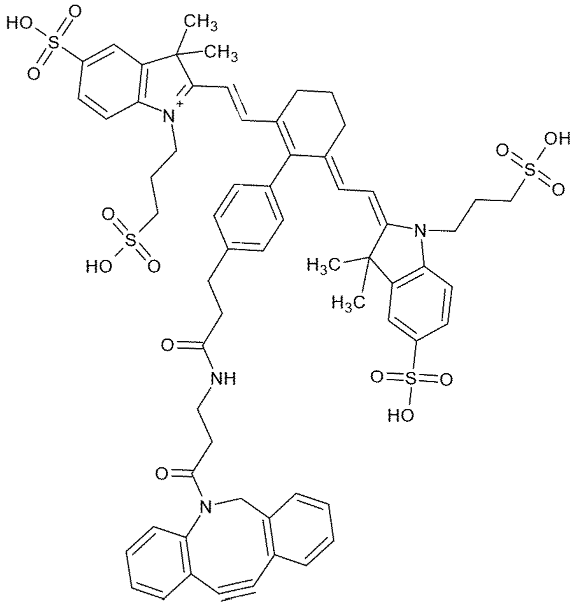

Formula III

Preferably, the fluorescent agent comprises a sulfo-cyanine moiety.

The non-fluorescent agent having an absorption wavelength of 650-900 nm, is a compound that has little or no intrinsic fluorescence and which can efficiently quench the fluorescence from a proximate fluorophore with little background. In an embodiment the non-fluorescent agent is a cyanine molecule. Cyanine molecules, also referred to as cyanine dyes, include compounds having two substituted or unsubstituted nitrogen-containing heterocyclic rings joined by a polymethine chain.

Preferably, the non-fluorescent agent comprises a sulfo-cyanine moiety.

In a preferred embodiment, the non-fluorescent agent is an agent wherein $R^1$ is chloro, $R^2$, $R^3$, $R^9$,$R^{10}$ are methyl; $R^4$ is H and $R^5$ is N-hydrocarbyl, preferably $N[(CH_2)_3SO_3Na]_2$;

$R^{11}$ and $R^{12}$ form a aromatic ring monosubsituted with sulfanato group; $R^6$, $R^7$, $R^{13}$, $R^{14}$ are H; $R^8$ is $(CH_2)$qFG where q is 3 and FG is sulfanato; $R^{15}$ is $(CH_2)_P$LN where p is 5 and LN is carboxyl; $R^{16}$ is H.

In another embodiment, the fluorescent agent and the non-fluorescent are the same agent, preferably wherein $R^1$ is

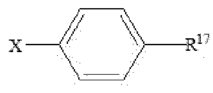

wherein X is O and $R^{17}$ is $SO_3Na$, $R^2$, $R^3$, $R^9$,$R^{10}$ are hydrocarbyl, preferably methyl, $R^4$, $R^5$, $R^{11}$, $R^{12}$ are H, $R^6$, $R^7$, $R^{13}$, $R^{14}$ are H, $R^8$ is $(CH_2)$qFG, where q is 4 and FG is sulfanato, $R^{15}$ is $(CH_2)$PLN where p is 5 and LN is carboxyl, $R^{16}$ is H.

The non-fluorescent agent may also be a quenching moiety for example BHQ3 (Biosearch), QC-1 (Li-COR.com), or particles comprising such compounds, for example gold nanoparticles and ferro-nanoparticles. In an embodiment, the reagent is a nanoparticle comprising a peptide as defined herein.

Examples of fluorescent agents that can be used with in present invention include, but are not limited to, Alexa Fluor® 660, Alexa Fluor® 680, Alexa Fluor® 700, Alexa 25 Fluor® 750 ATTO 680, ATTO 700, DY-647, DY-650, DY-673, DY-675, DY-676, DY-680, DY-681 , DY- 682, DY-690, DY-700, DY-701 , DY-730, DY-731 , DY-732, DY734, DY-750, DY-751 , DY-752, DY-776, DY-781 , DY-782, DY-831 , La Jolla Blue, Cy5, Cy5.5, Cy7, IRDye® 800CW, IRDye® 38, IRDye® 800RS, IRDye® 700DX, IRDye® 680, TF7WS, TF8WS, TideDye or TideQuencher, among others. "Alexa Fluor" dyes are available from Molecular Peptides Inc., Eugene, OR, U.S.A. (www.peptides.com). "ATTO" dyes are available from ATTO-tec GmbH, Siegen, Germany (www.atto-tec.com). "DY" dyes are available from Dyomics GmbH, Jena, Germany (www.dyomics.com). La Jolla Blue is available from Hyperion Inc. "Cy" dyes are available from Amersham Biosciences, Piscataway, NJ, U .S.A. (www.amersham.com)." IRDye® infrared dyes" are available from LI-COR® Bioscience, Inc. Lincoln, N E, U.S. A (www.licor.com).

Preferably, the fluorescent agent is selected from the group consisting of Cy7, Cy7.5, TF7WS, TF8WS and

IRDye800CW.

Preferably, the non-fluorescent agent having an emission wavelength of 650-900 nm is a cyanine moiety, preferably QC-1.

Most preferably, the reagent comprises a fluorescent agent selected from the group consisting of Cy7, Cy7.5, TQ7WS and IRDye800CW and a non-fluorescent agent having an emission wavelength of 650-900 nm being QC-1.

The reagent preferably comprises a fluorescent agent selected from the group consisting of Cy7, Cy7.5, TF7WS and IRDye800CW and a non-fluorescent agent having an emission wavelength of 650-900 nm being QC-1 and a cleavage site comprising at least three amino acids.

The bodily fluid sample is a human or animal bodily fluid sample, more preferably the bodily fluid is a human bodily fluid.

Preferably, the sample has a volume in the range of 10 μl to 3000 μL, more preferably, 50 μL to 2500 μL, even more preferably 100 to 2000 μL, yet more preferably 150 to 1500 μL and most preferably 200 to 1000 μL.

Preferably, the concentration of reagent in the sample receptacle after addition of the sample, and optionally a diluent, is in the range of 0.01 to 10 uM, preferably 0.02 to 8 uM, more preferably 0.05 to 5 uM, even more preferably 0.1 to 2.5 uM.

Preferably, step i) comprises the step of adding a diluent. Preferably, the diluent is an aqueous solution.

Preferably wherein the diluent comprises a buffering agent selected from the group consisting of MOPS, phosphate, citrate, HEPES TRIS-HCl, phosphate buffered saline. The preparation of aqueous solutions comprising buffering agents is known to the skilled person.

Preferably the diluent has a pH in the range of 5-9, more preferably in the range 5.5 to 8.5, even more preferably 5.75 to 8.25, yet more preferably 6 to 8.

Preferably, the volume ratio of diluent to sample is in the range of 0.1 to 100, more preferably 0.5 to 75, even more preferably in the range 1 to 50.

Preferably the sample is diluted in an assay buffer, preferably wherein the sample is diluted by a factor in the range of 1:10 to 1:10000, more preferably 1:100 to 1:1000.

Preferably, the assay buffer is capable of maintaining a pH in the range of 5-9, preferably about 6 to about 8, more preferably about 6.5 to about 7.5. preferably the buffer comprises HEPES, PIPES, Tris-Hydrochloride (Tris-HCl), phosphate, phosphate buffered saline, or MOPS. Preferably the buffer is selected from the group of phosphate buffer and phosphate buffered saline. Preferably the phosphate buffer comprises sodium and/or potassium ions.

Preferably, the buffer contains a detergent that is capable of lysing the cellular material in the bodily fluid sample.

Preferably, the buffer comprises one or more non-ionic detergents, selected from the group consisting of N-octyl- -D-glucopyranside, N- octyl- D-maltoside, ZWITTERGENT 3.14, deoxycholate; n-Dodecanoylsucrose; n- Dodecyl- -Dglucopyranoside; n-Dodecyl- -D-maltoside; n-Octyl- -D-glucopyranoside; n-Octyl-p-Dmaltopyranoside; n-Octyl-p-D-thioglucopyranoside; n- Decanoylsucrose; n-Decyl-p-D-maltopyranoside; n-Decyl-p-D-thiomaltoside; n- Heptyl- -D-glucopyranoside; n-Heptyl-p-D-thioglucopyranoside; n-Hexyl- -D- glucopyranoside; n-Nonyl-p-D-glucopyranoside; n-Octanoylsucrose; n-Octyl- -D- glucopyranoside; n-Undecyl- -D-maltoside; APO-10; APO12; Big CHAP; Big CHAP, Deoxy; BRIJ® 35; d2E5; d2E6; Ci2E8; Ci2E9; Cyclohexyl-nethyl-p-D- maltoside; Cyclohexyl-n-hexyl-p-D-maltoside; Cyclohexyl-n-methyl- -D-maltoside; Digitonin; ELUGENT™; GENAPOL® C-100; GENAPOL® X-080; GENAPOL® X-100; HECAMEG; MEGA-10; MEGA-8; MEGA-9; NOGA; NP-40; PLURONIC® F-127; TRITON® X-100; TRITON® X-I 14; TWEEN® 20; or TWEEN® 80 and mixtures thereof.

The buffer preferably comprises an ionic detergent selected from the group consisting of BATC, Cetyltrimethylammonium Bromide, Chenodeoxycholic Acid, Cholic Acid, Deoxycholic Acid, Glycocholic Acid, Glycodeoxycholic Acid, Glycolithocholic Acid, Lauroylsarcosine, Taurochenodeoxycholic Acid, Taurocholic Acid, Taurodehydrocholic Acid, Taurolithocholic Acid, Tauroursodeoxycholic Acid, TOPPA and mixtures thereof.

Preferably, the buffer comprises a zwitterionic detergent selected from the group consisting of amidosulfobetaines, CHAPS, CHAPSO, carboxybetaines, and methylbetaines.

Preferably, the buffer comprises an anionic detergent selected from group consisting of SDS, N-lauryl sarcosine, sodium deoxycholate, alkyl-aryl sulphonates, long chain (fatty) alcohol sulphates, olefine sulphates and sulphonates, alpha olefine sulphates and sulphonates, sulphated monoglycerides, sulphated ethers, sulphosuccinates, alkane sulphonates, phosphate esters, alkyl isethionates, sucrose esters and mixtures thereof.

In the present application, a buffering agent is a chemical species that is a capable of adjusting the pH of the buffer and/or sample combination.

Preferably, the sample of bodily fluid, is contacted with the reagent directly. The term "directly" as used herein means that the sample is not subjected to a centrifugation step or other sample clean up step prior to contact with the reagent.

Preferably, the sample of bodily fluid, is contacted with the reagent directly.

Optionally, the method comprises the step of centrifuging the sample can be centrifuged to separate aggregated bacteria matter and/or precipitated biological material from a supernatant. Preferably, centrifugation can be at a force in the

range of 1,000 g to 25,000 g, preferably, in the range of 3,000 g to 20,000 g, more preferably in the range of 3,700 g to 18,000 g. The centrifugation time is preferably in the range of 1 minute to 30 minutes, in the range of 2 to 20 minutes, more preferably in the range of 3 to 10 minutes. The supernatant or the aggregated bacterial matter cell resulting from centrifugation is preferably contacted with the reagent. In a preferred embodiment, the supernatant from centrifugation is preferably contacted with the reagent. In another preferred embodiment, the aggregated bacterial matter cell resulting from centrifugation is preferably contacted with the reagent.

Preferably, the method comprises the step of centrifuging the sample prior to contacting the sample with the reagent. For example, if there is some contamination of the sample with blood, it is desirable to centrifuge the sample prior to processing in the assay.

Optionally, after addition of the reagent to the sample the mixture is agitated. The mixture is preferably agitated by aspiration with a pipette, vortexing or shaking.

Preferably, the reagent is a peptide, protein or nanoparticle. Preferably, the reagent is a peptide. The peptide may be linked via a linker to a protein, nanoparticle or magnetic bead. The peptide preferably comprises between 3 and 10 amino acids, more preferably between 4 and 9 amino acids, even more preferably between 5 and 8 amino acids.

Preferably [b] comprises between 3 and 8 amino acids, more preferably between 3 and 7 amino acids, even more preferably between 3 and 6 amino acids.

The cleavage site preferably comprises of a limited number of amino acids, for example at least two, preferably at least three amino acids, more preferably at least four amino acids, even more preferably at least five amino acids.

Preferably, the cleavage site consists of between 2 and 5 amino acids, more preferably between 3 and 5 amino acids.

Preferably, [b] has the general formula $X_{aa1}$,$X_{aa2}$,$X_{aa3}$. In other words, the cleavage site preferably comprises three amino acids $X_{aa1}$,$X_{aa2}$,$X_{aa3}$ wherein

- $X_{aa1}$ is preferably a small, hydrophobic amino acid, more preferably $X_{aa1}$ is selected form the group consisting of alanine, glycine, leucine, valine, norleucine, norvaline, isoleucine, isovaline, alloisoleucine;
- $X_{aa2}$ is preferably a small or aromatic hydrophobic amino acid, preferably $X_{aa2}$ is selected from the group consisting of alanine, glycine, leucine, valine, norleucine, norvaline, isoleucine, isovaline, alloisoleucine and phenylalanine;
- $X_{aa3}$ is preferably selected form the group consisting of alanine, glycine, leucine, valine, norleucine, norvaline, isoleucine, isovaline, alloisoleucine, more preferably $X_{aa3}$ is selected form the group consisting of alanine, glycine, leucine, valine.

In certain preferred embodiments, $X_{aa1}$ is selected form the group consisting of alanine, valine, norleucine, norvaline, isoleucine, isovaline, alloisoleucine, $X_{aa2}$ is selected from the group consisting of alanine, leucine, valine, norleucine, norvaline, isoleucine, isovaline, alloisoleucine and phenylalanine; and $X_{aa3}$ is selected form the group consisting of alanine, leucine, valine.

Preferably, the cleavage site comprises three amino acids $X_{aa1}$,$X_{aa2}$,$X_{aa3}$ wherein

- $X_{aa1}$ is preferably a small, hydrophobic amino acid, more preferably $X_{aa1}$ is selected form the group consisting of alanine, glycine, leucine, valine, norleucine, norvaline, isoleucine, isovaline, alloisoleucine; more preferably alanine, leucine, valine, norleucine, norvaline, isoleucine, isovaline, alloisoleucine;
- $X_{aa2}$ is preferably a small or aromatic hydrophobic amino acid, preferably, $X_{aa2}$ is selected from the group consisting of alanine, glycine, leucine, valine, norleucine, norvaline, isoleucine, isovaline, alloisoleucine and phenylalanine; more preferably, $X_{aa2}$ is selected from the group consisting of alanine, leucine, valine, norleucine, norvaline, isoleucine, isovaline, alloisoleucine and phenylalanine;
- $X_{aa3}$ is preferably selected form the group consisting of alanine, leucine, valine, norleucine, norvaline, isoleucine, isovaline, alloisoleucine, more preferably $X_{aa3}$ is selected form the group consisting of alanine, glycine, leucine, valine,

wherein the cleavage site is cleaved by a bacterial biomarker wherein the bacterial biomarker is produced by a bacteria selected from the group consisting of *Enterococcus faecalis, Enterococcus faecium, Pseudomonas aeruginosa, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida glabrata, Candida tropicalis, and Candida albicans, more preferably* the causative agent (bacteria) is selected from the group consisting of *Bacillus spp, Staphylococcus spp, Streptococcus spp, Pseudomonas spp, Escherichia coli* and combinations thereof, most preferably from the group of *Bacillus spp and, Pseudomonas spp*

Preferably, the cleavage site comprises three amino acids $X_{aa1}$,$X_{aa2}$,$X_{aa3}$ wherein

- $X_{aa1}$ is preferably a small, hydrophobic amino acid, more preferably $X_{aa1}$ is selected form the group consisting of alanine, glycine, leucine, valine, norleucine, norvaline, isoleucine, isovaline, alloisoleucine; more preferably alanine, , leucine, valine, norleucine, norvaline, isoleucine, isovaline, alloisoleucine;
- $X_{aa2}$ is preferably a charged amino acid, preferably, $X_{aa2}$ is selected from the group consisting of aspartic acid, glutamic acid, lysine, arginine,
- $X_{aa3}$ is preferably selected form the group consisting of alanine, leucine, valine, norleucine, norvaline, isoleucine, isovaline, alloisoleucine, more preferably $X_{aa3}$ is selected form the group consisting of alanine, glycine, leucine, valine,

wherein the cleavage site is cleaved by a bacterial biomarker wherein the bacterial biomarker is produced by bacteria selected from the group consisting of *Enterococcus faecalis, Enterococcus faecium, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida glabrata, Candida tropicalis, and Candida albicans, more preferably* the causative agent (bacteria) is selected from the group consisting of *Staphylococcus spp, Streptococcus spp, Escherichia coli* and combinations thereof.

Preferably, [b] comprises an amino acid sequence selected from the group consisting of AAA, ALA, AAL, LAA, FAA, AFA, AAF, FGG, GFG, GGF, GGG, LGG, GLG, GGL and GGA. More preferably, [b] comprises an amino acid sequence selected from the group consisting of FGG, GFG, GGF, GGG, LGG, GLG, GGL and GGA. Yet more preferably, [b] comprises an amino acid sequence selected from the group consisting of AAA, ALA, AAL, LAA, FAA, AFA, AAF. Even more preferably, [b] comprises an amino acid sequence selected from the group consisting of FAA, AFA and AAF.

Preferably, [b] comprises an amino acid sequence selected from the group consisting of AVA, AAV, VAA, YAA, AYA, AAY, YGG, GFG, GGY, GGV, VGG, GVG, GAG and AGG. More preferably, [b] comprises an amino acid sequence selected from the group consisting of YGG, GFG, GGY, GGV, VGG, GVG, GAG and AGG. Yet more preferably, [b] comprises an amino acid sequence selected from the group consisting of AVA, AAV, VAA, YAA, AYA and AAY.

Preferably, [b] comprises an amino acid sequence selected from the group consisting of AEA, AAE, EAA, DAA, ADA, AAD, EGG, GEG, GGE, GGD, DGG, GDG, GRG and RGG. More preferably, [b] comprises an amino acid sequence selected from the group consisting of KGG, GKG, GGK, AKA, AAK, KAA, KAA, AKA and AAK.

Preferably, the mass ratio of [b] : ([a]+[c]) is at least 1:2, preferably the mass ratio of [b] : ([a]+[c]) is at least 1:3, more preferably the mass ratio of [b] : ([a]+[c]) is at least 1:4, even more preferably the mass ratio of [b] : ([a]+[c]) is at least 1:5.

Preferably, the reagent has the general formula [a]-[linker1]n-[b]-[-linker2]m-[c], wherein n is 0, 1 or 2, m is 0, 1 or 2, [linker1] and [linker2] are independently selected from the group of an optionally substituted hydrocarbyl group and a non-proteolytic hydrocarbyl group, preferably n is 0 or 1, m is 1 and linker2 is a non-proteolytic hydrocarbyl group, more preferably n is 0, m is 1 and linker2 is a non-proteolytic hydrocarbyl group.

Preferably, the linker is a non-proteolytic hydrocarbyl group.

Preferably, the linker is selected from the group consisting of beta-alaninyl, 4-aminobutyrl, 2-(aminoethoxy)acetyl, 3-(2-aminoethoxy)propanyl, 5-aminovaleryl, 6-aminohexyl, 8-amino-3,6-dioxaoctanyl and 12-amino-4,7,10-trioxadodecanyl, preferably 6-aminohexyl.

Preferably, the reagent has the general formula [a]-[$linker_1$]-[b]-[-$linker_2$]-[c] (II), wherein [$linker_1$] and [$linker_2$] are independently selected from the group of an optionally substituted hydrocarbyl group and a non-proteolytic hydrocarbyl group. Hydrocarbyl as used herein means optionally substituted C1-C6 alkyl. Preferably, the optionally substituted C1-C6 alkyl is substituted by O, N and S.

More preferably the reagent is of the general formula [a]-[linker1]$_n$-[b]-[linker2]$_m$-[c], wherein [b] is an amino acid sequence selected from the group consisting AAA, ALA, AAL, LAA, FAA, AFA, AAF, FGG, GFG, GGF, GGG, LGG, GLG, GGL and GGA and n is 1 and m is 1 and [linker1] and [linker2] are independently selected from the group consisting of beta-alaninyl, 4-aminobutyrl, 2-(aminoethoxy)acetyl, 3-(2-aminoethoxy)propanyl, 5-aminovaleryl, 6-aminohexyl

More preferably the reagent is of the general formula [a]-[b]-[linker]-[c], wherein b is an amino acid sequence selected from the group consisting of AAA, ALA, AAL, LAA, FAA, AFA, AAF, FGG, GFG, GGF, GGG, LGG, GLG, GGL and GGA, n is 0, m is 1 and [linker] is selected from the group consisting of beta-alaninyl, 4-aminobutyrl, 2-(aminoethoxy)acetyl, 3-(2-aminoethoxy)propanyl, 5-aminovaleryl, 6-aminohexyl.

More preferably the reagent is of the general formula [a]-[linker]-[b]-[c], wherein b is an amino acid sequence selected from the group consisting of AAA, ALA, AAL, LAA, FAA, AFA, AAF, FGG, GFG, GGF, GGG, LGG, GLG, GGL and GGA, n is 1 and m is 0 and [linker] is selected from the group consisting of beta-alaninyl, 4-aminobutyrl, 2-(aminoethoxy)acetyl, 3-(2-aminoethoxy)propanyl, 5-aminovaleryl, 6-aminohexyl.

Preferably, the method comprises the step of contacting the sample with a first reagent and a second reagent, wherein the first reagent has the general formula [a]-[b]-[c] (I) and [b] is a peptide comprising a cleavage site (b') and the second reagent has the formula [a]-[d]-[c] (III), wherein [d] is a peptide comprising a cleavage site (d'), wherein the cleavage site d' is different to the cleavage site b'.

An advantage of the method disclosed herein is that it enables a multiplexing approach, whereby different reagents comprising different cleavage sites can be combined in a single sample receptacle to enable detection of different bacterial biomarkers.

Preferably, wherein the bacterial biomarker is a bacterial membrane bound protease, bacterial membrane bound transpeptidase, intracellular bacterial protease or extracellular bacterial protease, more preferably the bacterial biomarker is a bacterial membrane bound transpeptidase or an extracellular bacterial protease.

Preferably, the method has a specificity for bacterial infection over host inflammation response.

Within the context of the present application "host inflammation response" means a host inflammation biomarker, for example a host inflammation protease. Within the context of the present application "specificity" for infection over host inflammation response means that ratio of fluorescence emission in the range 650-900 nm in the presence of a bacterial biomarker to the fluorescence emission in the range 650-900 nm in the presence of a host inflammation response is at least 1.05:1, preferably at least 1.5 to 1, more preferably at least 2:1, even more preferably at least 5:1, yet more preferably at least 10:1, even yet more preferably at least 25:1, most preferably at least 50:1.

Preferably the ratio of fluorescence emission in the range 650-900 nm in the presence of a bacterial biomarker to the fluorescence emission in the range 650-900 nm in the presence of a host inflammation response is in the range of 1.05:1 to 500:1, preferably 2:1 to 400:1, more preferably 5:1 to 300:1.

Preferably the ratio of fluorescence emission in the range 650-900 nm in a sample of bodily fluid that comprises a bacterial biomarker to the fluorescence emission in the range 650-900 nm in a sample of bodily fluid that does not comprise a bacterial biomarker, is in the range of 1.05:1 to 500:1, preferably 2:1 to 400:1, more preferably 5:1 to 300:1.

Preferably the ratio of fluorescence emission in the range 650-900 nm in a sample of bodily fluid that comprises a bacterial biomarker to the fluorescence emission in the range 650-900 nm in a sample of bodily fluid that does not comprise a bacterial biomarker, is in the range of 1.05:1 to 500:1, preferably 2:1 to 400:1, more preferably 5:1 to 300:1, wherein the bodily fluid sample is selected from the group consisting of the bodily fluid sample is selected from the group consisting of gingival fluid, amniotic fluid, urine, serous fluid, synovial fluid, peritoneal dialysis fluid, semen, sebum and cerebrospinal fluid, preferably wherein the bodily fluid is selected from the group consisting of synovial fluid, peritoneal dialysis fluid and cerebrospinal fluid, even more preferably wherein the bodily fluid is selected from the group consisting of synovial fluid and peritoneal dialysis fluid.

Preferably, the method has a specificity for bacterial infection of at least 70 %, preferably at least 75 %, more preferably at least 80 %, even more preferably at least 85%, yet more preferably at least 90%, most preferably at least 95%.

Within the context of the present application, the term "specificity percentage" is determined according to the formula:

$$\text{Specificity } \% = \frac{a}{b}\text{x}100$$

wherein a is the number of samples measured according to the method described herein which have a fluorescence emission in the range of 650-900 nm > threshold fluorescence emission in the range 650-900 nm;

b is the number of clinically positive samples for the target condition, wherein the clinically positive samples are determined to be positive for a condition by a relevant known reference method. Typically, a reference method for the determination of infection in a bodily fluid sample is the relevant clinical guidelines known to the skilled person.

The threshold fluorescence emission is value of the fluorescence emission that has been determined to be the cut-off value for the discrimination of a positive sample from a negative sample. Typically, the threshold fluorescence is determined according to the formula

$$\text{Threshold} = \text{y x LOD}$$

where y is an integer in the range of 1 and 10, preferably y is in an integer in the range of 2 and 9, more preferably y is an integer in the range 3 and 8, even more preferably y is an integer in the range of 4 and 7; and

LOD is the limit of detection, where the LOD is determined as the lowest detectable amount of the target analyte. Determination of the LOD is well within the capabilities of the skilled person. as described by David A Armbruster and Terry Pry, Clin Biochem Rev. 2008 Aug 29Suppl 1/ S49-S52.

In certain embodiment, the threshold is determined according to the formula

$$\text{Threshold} = \text{LOB} + \text{LOD}$$

where LOB is the mean blank fluorescence emission ; and

LOD is the limit of detection, where the LOD is determined as the lowest detectable amount of the target analyte. The skilled person understands that the threshold is determined for a particular bodily fluid. In other words, the skilled person understands that the reagent will have different threshold values in, for example, peritoneal dialysis fluid and synovial fluid. The skilled person can determine the threshold using known methods. Determination of the LOD is well within the capabilities of the skilled person. as described by David A Armbruster and Terry Pry, Clin Biochem Rev. 2008 Aug 29Suppl 1/ S49-S52.

In certain embodiment, the threshold is determined according to the formula

$$\text{Threshold} = \text{LOB} + 1.645(\text{standard deviation low concentration sample})$$

where LOB is the mean blank fluorescence emission; and LOD is the limit of detection, where the LOD is determined as the lowest detectable amount of the target analyte. Determination of the LOD is well within the capabilities of the skilled person. as described by David A Armbruster and Terry Pry, Clin Biochem Rev. 2008 Aug 29Suppl 1/ S49-S52

Preferably the method has a selectivity for gram positive bacteria. Within the context of the present application "selectivity" for gram positive bacteria means that ratio of fluorescence emission in the range 650-900 nm in the presence of a bacterial biomarker from gram positive bacteria to the fluorescence emission in the range 650-900 nm in the presence of a non-gram positive inflammation biomarker is at least 1.05:1, preferably at least 1.5 to 1, more preferably at least 2:1, even more preferably at least 5:1, yet more preferably at least 10:1, even yet more preferably at least 25:1, most preferably at least 50:1.

Preferably the method has a selectivity for gram negative bacteria. Within the context of the present application "selectivity" for gram negative bacteria means that ratio of fluorescence emission in the range 650-900 nm in the presence of a bacterial biomarker from gram negative bacteria to the fluorescence emission in the range 650-900 nm in the presence of a non-gram negative inflammation biomarker is at least 1.05:1, preferably at least 1.5 to 1, more preferably at least 2:1, even more preferably at least 5:1, yet more preferably at least 10:1, even yet more preferably at least 25:1, most preferably at least 50:1.

Within the context of the present application, "specificity" and "selectivity" are used interchangeably.

Preferably, the method has a specificity for gram positive bacteria of at least 70 %, preferably at least 75 %, more preferably at least 80 %, even more preferably at least 85%, yet more preferably at least 90%, most preferably at least 95%.

Preferably wherein the method has a specificity for gram negative bacteria of at least 70 %, preferably at least 75 %, more preferably at least 80 %, even more preferably at least 85%, yet more preferably at least 90%, most preferably at least 95%.

Preferably, the method detects infection wherein the bacteria (causative agent) is selected from the group consisting of *Enterococcus faecalis, Enterococcus faecium, Pseudomonas aeruginosa, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida glabrata, Candida tropicalis, and Candida albicans, more preferably* the causative agent (bacteria) is selected from the group consisting of *Bacillus spp, Staphylococcus spp, Streptococcus spp, Pseudomonas spp, Escherichia coli* and combinations thereof, most preferably from the group of *Bacillus spp, Staphylococcus spp, and, Pseudomonas spp.* The cleavage site is preferably specific for a bacterial biomarker is selected from the group consisting of *Enterococcus faecalis, Enterococcus faecium, Pseudomonas aeruginosa, Staphylococcus aureus, Staphylococcus epidermidis, Staphylococcus lugdunensis, Staphylococcus warneri, Staphylococcus capitis, Staphylococcus caprae, Streptococcus mitis, Streptococcus oralis, Streptococcus agalactiae, Streptococcus anginosus, Streptococcus gordonii, Escherichia coli, Propionibacterium acnes, Proteus mirabilis, Granulicatella adjacens, Acinetobacter baumannii, Abiotrophia defective, Corynebacterium striatum, Corynebacterium minutissimum, Parvimonas micra, Candida parapsilosis, Candida glabrata, Candida tropicalis, and Candida albicans, more prefererably* the causative agent (bacteria) is selected from the group consisting of *Bacillus spp, Staphylococcus spp, Streptococcus spp, Pseudomonas spp, Escherichia coli* and combinations thereof, most preferably from the group of *Bacillus spp, Staphylococcus spp, and, Pseudomonas spp.*

The cleavage site is preferably specific for a bacterial biomarker for bacteria selected from the group consisting of *Bacillus spp, Staphylococcus spp, Streptococcus spp, Pseudomonas spp, Escherichia coli* and combinations thereof. The cleavage site is preferably specific for a bacterial biomarker for bacteria from the group consisting of *Bacillus cereus, Staphylococcus aureus, Streptococcus pyogenes, Pseudomonas aeruginosa, Escherichia coli* and combinations thereof, most preferably from the group of *Bacillus spp, Staphylococcus spp, and Pseudomonas spp.*

Preferably, the cleavage site is specific for a bacterial biomarker for a bacteria species selected from the group consisting of *Bacillus cereus, Staphylococcus aureus, Streptococcus pyogenes, Pseudomonas aeruginosa, Escherichia coli* and combinations thereof, preferably wherein the cleavage site is specific for *Pseudomonas aeruginosa and Bacillus cereus,* more preferably wherein the cleavage site is specific for *Pseudomonas aeruginosa.*

Preferably, the cleavage site is specific for a bacterial biomarker for a bacteria species selected from the group consisting of *Staphylococcus aureus, Pseudomonas aeruginosa, Escherichia coli* and combinations thereof, preferably wherein the cleavage site is specific for *Pseudomonas aeruginosa and Staphylococcus aureus,* more preferably wherein the cleavage site is specific for *Staphylococcus aureus.*

In a particularly preferred embodiment, the sample is not subjected to a bacterial cell enrichment step prior to contacting the sample with the reagent. By "not subjected to a bacterial cell enrichment step" means there is not a step of incubating (ex vivo) the sample at a temperature in the range of 10-37°C for a period time, for example for a number of hours in order to increase the optical density (turbidity, measured absorbance at 600 nm) of the sample.

Preferably, the sample is not subjected to an ex vivo step of bacterial enrichment in the temperature range of 20 to 40°C either prior to, or after, being contacted with the reagent.

The step of monitoring the increase in fluorescence in step iii) is preferably carried out in a detector adapted to receive a container comprising the sample and reagent. Preferably, the sample is contacted with the reagent in a container and the fluorescence emitted by the reagent is monitored by a handheld or benchtop fluorescence spectrometer adapted to receive the container. The detector preferably provides a readout in relative fluorescent units (RFU). The user can compare the readout to a control and a relative increase in RFU between the control and the sample is indicative of bacterial infection.

Preferably, step of monitoring the increase in fluorescence in step iii) is preferably carried out by contacting a sample with the reagent and the fluorescence emitted by the reagent is monitored by a benchtop fluorescence spectrometer, preferably a plate reader. Preferably, the sample is contacted with the reagent, preferably in a sample tube, and the mixture comprising sample and reagent transferred to a multi-well plate, preferably a 96 well plate.

The increase in fluorescence (emission) is optionally detected by use of any of the following devices: CCD cameras, video cameras, photographic film, laser-scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using a flow cytometer, examination of the sample optionally includes sorting portions of the sample according to their fluorescence response.

Preferably, the reagent is not attached to a surface. It has been found that by providing the reagent in a form that is not covalently bound to a surface, non-specific interactions are unexpectedly reduced.

Preferably, the reagent in step i) is in the form of a lyophilized bead comprising a carbohydrate.

Preferably, the bead comprises 15 to 95 wt.% by weight of the bead carbohydrate, preferably 20 to 90 wt.%, more preferably 25 to 85 wt.%, yet more preferably 30 to 80 wt.% carbohydrate, by weight of the bead carbohydrate.

Preferably, the carbohydrate is selected from the group consisting of monosaccharide, disaccharide, polysaccharide and combinations thereof, the monosaccharide having the general formula $(CH_2O)_n$.

In a second aspect, there is provided a lyophilized bead according to claim 13.

It has been found that formulating the reagent as a lyophilized bead with a carbohydrate has the unexpected advantage of increasing clinical performance of the reagent. Surprisingly, the ratio of a fluorescent emission at wavelengths in the range of 650-900 nm in the presence of a bacterial biomarker to the fluorescent emission at wavelengths in the range of 650-900 nm in the absence of a bacterial biomarker increases when the reagent is in the form of a carbohydrate comprising lyophilized bead.

Without wishing to be bound by theory, it is postulated that the inclusion of a carbohydrate in the lyophilized bead minimizes hydrophobic interactions between the reagent molecules in the bead such that an improved dequenching is observed, which in turn leads to an improved ratio in the presence and absence of bacterial biomarker, and thus to improved clinical performance.

The lyophilized bead typically has a size of in the range of 1-5 mm, preferably 2 to 4 mm.

Preferably, the bead comprises 5 to 95 wt.% by weight of the bead carbohydrate, preferably 10 to 90 wt.%, more preferably 15 to 85 wt.%, yet more preferably 20 to 80 wt.% carbohydrate, by weight of the bead carbohydrate.

The carbohydrate according to the invention is selected from the group consisting of monosaccharide, disaccharide, polysaccharide and combinations thereof. The carbohydrate is preferably a monosaccharide, disaccharide or polysaccharide. The identity of the carbohydrate can be selected by the skilled person by observing the relative activity of the lyophilized bead in the method disclosed herein. In certain embodiments, preferably a combination of carbohydrates may be used..

Preferably, the monosaccharide is selected from the group consisting of glucose, fructose, galactose and combinations thereof. Monosaccharides have the general formula $(CH_2O)_n$.

Monosaccharides are classified according to three different characteristics: the location of their carbonyl group, the number of carbon atoms they contain, and their chiral property. If the carbonyl group is an aldehyde, the monosaccharide is an aldose. If the carbonyl group is a ketone, the monosaccharide is a ketose. Monosaccharides with three carbon atoms are called trioses and these are the smallest monosaccharides, such as dihydroxyacetone and d- and l-glyceraldehyde. Those composed of four carbon atoms are called tetroses, those with five carbons are called pentoses, those of six carbons are hexoses, and so on. Other minor monosaccharides include mannose, galactose, xylose, and arabinose. The most commonly detected pentoses are arabinoses and xyloses.

Preferably the disaccharide is selected from the group consisting of sucrose, maltose, lactose, trehalose and combinations thereof. Disaccharides consist of two monosaccharide units, linked together with glycosidic bonds in the $\alpha$ or $\beta$ orientation. Preferably, the disaccharide is sucrose. Sucrose consists of a molecule of $\alpha$-glucose and $\beta$-fructose linked together. Preferably, the disaccharide is lactose. Lactose consists of galactose and glucose linked by a $\beta$-1,4-glycosidic bond. Preferably, the disacharide is maltose. Maltose is typically produced by partial hydrolysis of starch and consists of two glucose units linked by an $\alpha$-1,4-glycosidic bond.

Preferably, the polysaccharide is selected from the group consisting of alginate, chitosan, hyaluronic acid, cellulose derivatives, dextran and combinations thereof. Without wishing to be bound by theory, the presence of a polysaccharide carbohydrate is thought to stabilize the intramolecular interactions between the reagent molecules in the lyophilized bead such that the optimum quenching is obtained in the lyophilized bead.

Preferably, the polysaccharide is dextran. Dextran is a polymer of anhydroglucose. Typically, dextran comprises approximately 95% alpha-D-(1-6) linkages, with the remaining a(1-3) linkages accounting for the branching of dextran. Typically, the average branch length is in the range of 1 to 50, more preferably 5 to 40, even more preferably 10 to 30. Preferably, the average branch less is less than three glucose units.

Preferably, the molecular weight (MW) of dextran is in the range of 2000 to 500 million. Typically, lower MW dextrans will exhibit slightly less branching and have a more narrow range of MW distribution. Dextrans with MW greater than 10,000 typically behave as if they are highly branched. As the MW increases, dextran molecules attain greater symmetry. Dextrans with MW of 2,000 to 10,000 dextran molecules exhibit the properties of an expandable coil. At MWs below 2,000 dextran is more rod-like. Preferably the molecular weight of dextran is in the range 2000 to 20000, more preferably in the range of 3000 to 15000, even more preferably in the range of 4000 to 10000.

The MW of dextran is measured by methods well known to the skilled person, which many include but are not limited to low angle laser light scattering, size exclusion chromatography, copper-complexation, and anthrone reagent colorimetric reducing-end sugar determination and viscosity.

Preferably, step i) of the method described herein comprises contacting a sample of bodily fluid, preferably a human bodily fluid, with the reagent in a sample receptacle wherein the reagent is a lyophilized bead as described herein.

Preferably, in step i) the reagent is provided as a lyophilized bead in a tube. In some embodiments, the lyophilized bead is dissolved in a diluent to provide a reagent in a solution of diluent in a suitable receptacle and then the reagent in the solution of diluent is contacted with the sample. Preferably, the diluent is an aqueous solution. Preferably the aqueous solution comprises water and optionally one or more buffering agents and/or pH adjustment agents.

The embodiments described herein with regards to the method, apply mutatis mutandis to the embodiments described herein with regards to the lyophilized bead.

In a third aspect, there is provided a kit for in vitro diagnosis of infection in bodily fluid samples, comprising:

a) a container comprising a lyophilized bead as described above,:
b) a set of instructions for carrying out the method defined herein,
c) optionally a container containing a diluent.

Preferably, the reagent and the diluent are present in two compartments of a container, preferably wherein the compartments are separated by a pressure activatable membrane.

The preferred embodiments for the method described herein apply mutatis mutandis to the kit described herein.

The preferred embodiments for the lyophilized bead described herein apply mutatis mutandis to the kit described herein.

Preferably, the kit is for in vitro diagnosis of periprosthetic joint infection, peritoneal dialysis-related peritonitis and/or cerebrospinal associated infection, comprising:

a) a container comprising a lyophilized bead as described herein,
b) a set of instructions for carrying out the method defined herein

The inventors have found that by providing a system that comprises a device adapted to receive a container for receiving a sample of synovial fluid, peritoneal fluid or cerebrospinal fluid and contacting the sample with the reagent defined elsewhere herein, a rapid means of identifying an infection in said sample is provided.

Preferably, the system is a point-of-care system or a patient beside system.

Preferably, the system comprises

a) a container comprising a lyophilized bead as described herein,

b) a device adapted to receive the container and monitor the fluorescence signal emitted from the reagent in the presence of a sample of synovial fluid, peritoneal fluid or cerebrospinal fluid with the reagent, wherein the system is adapted to carry out a method described herein.

The preferred embodiments for the method described herein apply mutatis mutandis to the system described herein.

The present invention has been described above with reference to a number of exemplary embodiments.

## Examples

### Example 1

Peptide 1: Peptide cleavage site: AFA; a fluorescent agent: IRDye 800CW (Licor Bioscience, USA); non-fluorescent agent: QC-1 (Licor Bioscience, USA).

A control peptide (A) was used having the same fluorescent agent and non- fluorescent agent as (1); Peptide cleavage site: ADA.

Biological samples

Example 1.1

Composition of model synovial fluid:

- 2 mg/mL hyaluronic acid
- 25 mg/mL bovine serum albumin
- 4 mg/mL nutrient broth powder (Difco no. 234000)
- Prepared in phosphate buffered saline, pH 7.1

Example 1.2

Composition of model cerebrospinal fluid: 150 mM Na, 3.0 mM K, 1.4 mM Ca, 0.8 mM Mg, 1.0 mM P, 155 mM Cl (obtained from BiolVT).

Example 1.3

Composition of peritoneal fluid: DIANEAL (2.27%) CAPD Solution 2.5L (Baxter).

Bacteria

The following strains of bacteria were inoculated in the model synovial fluid, CSF and peritoneal fluid supplemented with a general purpose medium for growth:

- Pseudomonas aeruginosa (Ex 1) (gram negative)
- Bacillus cereus (Ex. 2) (gram positive)

Inoculation took place at three levels (CFU/mL): low ($1x10^1$-$1x10^3$), medium ($1x10^4 1x10^6$) and high ($1x10^7$-$1x10^9$) in 5 mL bottles.

24 hours after inoculation (as a model for a 24 hour post-operative clinical situation) a 250 uL sample of each culture was placed in an Eppendorf tube. Peptide 1 or Peptide A was added to a final concentration of 5 uM. The Eppendorf tube was placed in a DeNiro NIR Fluorimeter (Detact Diagnostics BV, The Netherlands), 24 hours after inoculation as a model for a 24 hour post-operative clinical situation.

The RFU for the control sample (peptide only) was subtracted from each measurement. Above baseline RFU measurement is indicated by (+). No change relative to the baseline is indicated with (-).

Table 1: 24 hours after inoculation with *P. aeruginosa*

| | | Peptide 1 | | | Peptide A | | |
|---|---|---|---|---|---|---|---|
| | | $1x10^3$ | $1x10^4$-$1x10^6$ | $1x10^7$-$1x10^9$ | $1x10^3$ | $1x10^4$-$1x10^6$ | $1x10^7$-$1x10^9$ |
| 1.1 | Synovial fluid | +++ | +++ | +++ | - | - | - |
| 1.2 | CSF | ++ | ++ | ++ | - | - | - |
| 1.3 | Peritoneal fluid | +++ | +++ | +++ | - | - | - |

Table 2: 24 hours after inoculation with *Bacillus cereus*

| | | Peptide 1 | | | Peptide A | | |
|---|---|---|---|---|---|---|---|
| | | $1x10^3$ | $1x10^4$-$1x10^6$ | $1x10^7$-$1x10^9$ | $1x10^3$ | $1x10^4$-$1x10^6$ | $1x10^7$-$1x10^9$ |
| 2.1 | Synovial fluid | ++ | ++ | ++ | - | - | - |
| 2.2 | CSF | + | + | + | - | - | - |
| 2.3 | Peritoneal fluid | + | + | + | - | - | - |

**Example 2**

Location of bacterial biomarker

The location of the bacterial biomarker in Ex. 1.1 and Ex.2.1 was determined by measuring the fluorescence activity (RFU) of the bacterial cultures, concentrated cells (following centrifugation) and supernatant (following centrifugation).

Table 3: RFU activity fractions

| **Example** | **Culture** | **Concentrated cells** | **supernatant** |
|---|---|---|---|
| **P. aeruginosa** | 19803 | 878 | 19000 |
| **B. cereus** | 6363 | 300 | 5838 |

**Example 3**

Peptide 2: Peptide cleavage site: ALA; fluorescent agent: IRDye 800CW (Licor Bioscience, USA); non-fluorescent agent: QC-1 (Licor Bioscience, USA)
Peptide B: Peptide cleavage site: AEA; fluorescent agent EDANS; non-fluorescent agent DABCYL.

The detection efficiency of Peptide 2 was compared to a Peptide B.
Example 1.1 and 2.1 were repeated using Peptide 2 and B. Detection took place using a UV-spectrophotometer for Peptides B and C.

Table 4: Peptide detection efficiency

| Peptide | Ratio [b]:([a]+[c]) | *P. aeruginosa* inoculation $1x10^4$-$1x10^6$ | *B. cereus* inoculation $1x10^4$-$1x10^6$ |
|---|---|---|---|
| 2 | 1:11 | +++ | ++ |
| B | 1:1.6 | - | - |

**Example 4**

Peritoneal dialysis fluid from a patient undergoing automatic peritoneal dialysis overnight, was collected after overnight dialysis. The dwell time of the dialysis fluid is approx. 3 hours. A sample of the waste fluid was collected and spiked with a culture of a clinical isolate of *Pseudomonas aeruginosa.* The clinical isolate had been collected from a patient

with clinical (bacterial) peritonitis.

The level of spiking done at 2 levels: high level consistent with that of a cloudy waste bag (x$10^8$ CFU/mL) and low level (x$10^5$ CFU/ml) consistent with a clinically relevant amount of bacteria that would lead to infection if not treated.

Table 5: Sample Composition

| | Positive sample | Negative sample |
|---|---|---|
| Peritoneal fluid volume | 95 μL | 20 μL |
| Bacterial culture volume* | 95 μL | 0 μL |
| Reagent volume | 10 μL | 10 μL |
| Final volume in assay tube | *200 μL* | *200 μL* |
| * bacterial culture either at x$10^8$ or x$10^5$ CFU/mL, optionally diluted with sodium phosphate buffer 200 mM, pH 8.75 | | |

The reagent (Peptide 3) was synthesised by solid phase peptide synthesis. TQ7WS and TF7WS were purchased from AATBIO (Sunnyvale, CA, USA)amide and PEG1 is Amino-PEG1-acid purchased from BroadPharm (San Diego, CA, USA). Peptide 3 was used at a final concentration in assay tube was 1 uM.

Peptide 3:

N-terminus: TQ7WS
C-terminus:TF7WS
Cleavage site: AFA
Linker: PEG1

Samples were analysed on a TECAN SPARK plate reader using Greiner 96 Flat Black plates and the following settings:

- Automatic mirror
- Z position = 20 000
- Gain = 175
- Shaking 10 s, orbital
- Kinetic loop :
- Duration : 30 min
- Interval type : Fixed 5 min
- Fluorescence intensity :
- Mode : Top
- Excitation wavelength : 756 nm Bandwidth : 5
- Emission wavelength : 780 nm Bandwidth : 5

A threshold of 15% relative activity was set for a positive outcome. The threshold was determined as the limit of the blank. The limit of the blank was determined by measuring the fluorescence emission of peptide 3 in phosphate buffer, pH 8.

The ratio of the fluorescence emission intensity (FI) at 780 nm of the positive sample to the negative sample is reported in the table below.

Table 6: Sample Composition

| Example | Bacteria | Inoculation level | Ratio FI positive: negative |
|---|---|---|---|
| 4.A | None (negative control) | none | - |
| 4.1 | P. aur | Low | 4 |
| 4.2 | P. aur | High | 10 |
| 4.3 | S. aureus | Low | 3.3 |

(continued)

| Example | Bacteria | Inoculation level | Ratio FI positive: negative |
|---|---|---|---|
| 4.4 | S. aureus | High | 10 |
| 4.5 | P. aur + S. aur | Low | 6.6 |
| 4.6 | P. aur + S. aur | High | 8 |

**Example 5**

Briefly, a culture of *S. aureus* and *P. aeruginosa* were prepared and used to spike human synovial fluid (Innovate Research, Novi, MI, USA). The samples were spiked to a CFU/mL in the range of. $1x10^8$ - $1x10^{10}$, which is representative of the bacterial load in infected synovial fluid in periprosthetic joint infection.

Samples were measured 5 minutes after spiking with bacterial culture. Samples were measured using the DeNIRO fluorometer.

The composition of the samples used is shown below in Table 2

Table 7: Sample Composition

| | Positive sample | Negative sample |
|---|---|---|
| Buffer volume | 130 μL | 130 μL |
| Synovial fluid volume | 20 μL | 20 μL |
| Bacterial culture volume | 40 μL | 0 μL |
| Water volume | 0 μL | 40 μL |
| Reagent volume | 10 μL | 10 μL |
| Final volume in assay tube | *200 μL* | *200 μL* |

Positive samples gave a ratio of the fluorescence emission intensity (FI) at 780 nm of the positive sample to the negative sample of more than 1.2, whereas the negative samples gave a relative activity below 1 at 5 minutes.

**Example 6**

The effect of linkers on the activity of the reagents with an AFA motif was determined in peritoneal dialysate fluid. The composition of the reagents is shown in Table 8. The reagents 6.1-6.5 were screened against a broad-spectrum bacterial protease (P5380, Sigma Aldrich, 4 nM final concentration) as an infection model. A sample of peritoneal dialysate fluid was diluted 50% with sodium phosphate buffer, pH8, mixed with the reagent to a final concentration of peptide of 1 uM in an Eppendorf tube and 200 uL transferred to a microwell plate (Greiner, black, chimney flat bottomed well plate) and the fluorescent emission monitored using a TECAN SPARK plate reader.

Dequenching efficiency was determined by measuring the fluorescent emission intensity at either 780 nm (TF7WS) or 796 nm (IRDye800CW) in the absence ($FI^1$) and presence of protease ($FI^2$) and determining the percentage change in intensity using the equation: $([(FI^2 - FI^1)/ FI^1]*100$

Table 8: Results in peritoneal fluid

| | Dye | Linker | Linker | Quencher | Dequenching efficiency (%) PF[1] | Ratio linker: no linker |
|---|---|---|---|---|---|---|
| 6.1 | TF7WS | None | None | TQ7 | 170 | 1 |
| 6.2 | TF7WS | None | PEG1 | TQ7 | 400 | 2.4 |
| 6.3 | TF7WS | PEG1 | PEG1 | TQ7 | 299 | 1.8 |
| 6.4 | Idye800CW | None | None | QC-1 | 67 | 1 |
| 6.5 | IRDye800CW | None | PEG1 | QC1 | 119 | 1.8 |
| [1] *n* = 12 | | | | | | |

Introduction of one or two PEG1 linkers lead to an unexpected improvement in the clinical performance of the

reagent.

**Example 7**

The effect of linkers on the activity of the reagents with an AFA motif was determined in synovial fluid. The composition of the reagents is shown in Table 8. The reagents 7.1-7.5 were screened against a broad-spectrum bacterial protease (P5380, Sigma Aldrich 4 nM final concentration) as an infection model in synovial fluid. A sample of synovial fluid was diluted 25% with sodium phosphate buffer, pH8, mixed with the reagent to a final concentration of peptide of 1 uM in an Eppendorf tube and 200 uL transferred to a microwell plate (Greiner, black, chimney flat bottomed well plate) and the fluorescent emission monitored using a TECAN SPARK plate reader The concentration of peptide was 1 uM. The concentration of the protease was 4 nM.

Dequenching efficiency was determined by measuring the fluorescent emission intensity at either 780 nm (TF7WS) or 796 nm (IRDye800CW) in the absence ($FI^1$) and presence of protease ($FI^2$) and determining the percentage change in intensity using the equation: $([(FI^2 - FI^1)/ FI^1]*100$

Table 8: Results in synovial fluid

| | Dye | Linker | Linker | Quencher | Dequenching efficiency (%) SF[1] | Ratio linker: no linker |
|---|---|---|---|---|---|---|
| 7.1 | TF7 | None | None | TQ7 | 103 | 1 |
| 7.2 | TF7 | None | PEG1 | TQ7 | 265 | 2.6 |
| 7.3 | TF7 | PEG1 | PEG1 | TQ7 | 150 | 1.5 |
| 7.4 | Idye800CW | None | None | QC-1 | 35 | 1 |
| 7.5 | IRDye800CW | None | PEG1 | QC1 | 76 | 2.2 |
| [1] *n* = 12 | | | | | | |

Introduction of one or two PEG1 linkers lead to an unexpected improvement in the clinical performance of the reagent.

**Example 8**

Peptide 1 was formulated as a freeze-dried lyophilized bead by freeze drying the peptide in the presence of a carbohydrate. Briefly, 25 ug peptide 1 was dissolved in 300 uL PBS, pH 7.4 and added to 300 uL PBS, pH 7.4 containing 20% carbohydrate. 24 beads were dispensed and freeze dried (74 hours, primary temperature -45°C).

Per excipient, 2 measurements were taken using 2 identical beads. Firstly, in a 0.5 mL microcentrifuge tube, a lyophilized bead was dissolved in 300 uL sterile water at room temperature to prepare the negative sample, the tube was transferred to a DeNIRO® Fluorometer (Detact Diagnostics, Groningen, The Netherlands) and fluorescent emission intensity at 780 nm (TF7WS) recorded. Secondly, in a 0.5 mL microcentrifuge tube, a lyophilized bead was dissolved in 300 uL a solution of subtilisin (4 nM) in sterile water to prepare the positive sample. The mixture was briefly vortexed, incubated at 21°C for 15 minutes and then fluorescent emission intensity at 780 nm (TF7WS) measured using the DeNIRO® Fluorometer.

The ratio of the fluorescence emission intensity (FI) at 780 nm of the positive sample to the negative sample is reported in the table below.

Table 9: Results

| | Carbohydrate | FI Negative | FI Positive | Ratio RFU2:RFU1 |
|---|---|---|---|---|
| **7 A** | None | 12101 | 16451 | 1.4 |
| **7.1** | Dextran[1] | 6794 | 27044 | 4 |
| **7.2** | Trehalose[2] | 13293 | 23717 | 2 |
| **7.3** | Lactose[3] | 11725 | 24244 | 2.1 |
| **7.4 (Comparative)** | Mannitol[4] | 11528 | 16888 | 1.5 |
| [1] Dextran: Sigma cat 31388; [2]Trehalose: Life Sciences cat TDH033;<br>[3] Lactose: Sigma cat L3750; [4] Mannitol: Sigma cat M4125 | | | | |

Table 9 shows that all formulations containing carbohydrate gave an unexpected improvement in relative activity. Surprisingly, lyophilized beads comprising a polysaccharide, dextran, showed an unexpected stabilization of the background (lowest negative FI) and the highest ratio of positive to negative samples at 780 nm.

**Example 10**

Lyophilized beads comprising dextran according to Example 9 were dissolved 200 uL of bodily fluid (peritoneal dialysis fluid and synovial fluid as shown in Table 11). The bacterial cultures were prepared as per Example 4.

Table 10: Sample composition

| | **Positive sample** | **Negative sample** |
|---|---|---|
| Buffer volume | 140 μL | 140 μL |
| Bodily fluid volume | 20 μL | 20 μL |
| Bacterial culture volume | 40 μL | 0 μL |
| Water volume | 0 μL | 40 μL |
| Final volume in assay tube | *200 μL* | *200 μL* |

Table 11: Results

| | **Clinical Sample** | **Positive sample** | **Negative sample** |
|---|---|---|---|
| **8.1** | Peritoneal dialysis fluid | > threshold | < threshold |
| **8.2** | Synovial fluid | > threshold | < threshold |

**Comparative Example**

Clinical samples of peritoneal fluid and synovial fluid were spiked with a culture of S. aureus as per Example 4 and 5 and subjected to an acidification step as described by Example 1 of WO2018/224561 prior to determining the dequenching efficiency. 1 mL of clinical sample was adjusted to pH 4.6 by addition of 10% acetic acid. Solutions were kept at room temperature for 15 minutes. 0.1 mL 1 M sodium acetate buffer was added and the samples were centrifuged for 30 minutes at 4500g. When the samples were analysed as per Example 10, no measurable activity above the threshold was found.

**Claims**

**1.** A method for in vitro diagnosing infection in bodily fluid samples using a reagent having the general formula [a]-[b]-[c] (I), wherein:

[a] is a fluorescent agent having an emission wavelength of 650-900 nm,
[c] is a non-fluorescent agent having an absorption wavelength of 650-900 nm, for quenching said emission of said fluorescent agent,
[b] is a peptide comprising a cleavage site, the cleavage site being specific for a bacterial biomarker, wherein the bacterial biomarker is a bacterial membrane bound protease, bacterial membrane bound transpeptidase, intracellular bacterial protease or extracellular bacterial protease, the reagent being provided in the form of a lyophilized bead comprising a carbohydrate, chosen from the group, consisting of monosaccharide, disaccharide, polysaccharide and combinations thereof, the monosaccharide having the general formula $(CH_2O)_n$, wherein the method comprises the steps of:

i) contacting a sample of bodily fluid, preferably a human bodily fluid, with the reagent in a sample receptacle, and
ii) monitoring the fluorescence emission in the range of 650-900 nm from the sample in the receptacle in step i), wherein an increase in fluorescence emission in the range of 650-900 nm is indicative for the presence of infection in the bodily fluid sample.

2. The method according to claim 1, wherein the bodily fluid sample is selected from the group consisting of amniotic fluid, urine, serous fluid, synovial fluid, peritoneal dialysis fluid, semen, sebum and cerebrospinal fluid, preferably wherein the bodily fluid is selected from the group consisting of synovial fluid, peritoneal dialysis fluid and cerebrospinal fluid, even more preferably wherein the bodily fluid is selected from the group consisting of synovial fluid and peritoneal dialysis fluid.

3. The method according to claim 1 or 2, wherein the mass ratio of [b] : ([a]+[c]) is at least 1:2, preferably the mass ratio of [b] : ([a]+[c]) is at least 1:3, more preferably the mass ratio of [b] : ([a]+[c]) is at least 1:4, even more preferably the mass ratio of [b] : ([a]+[c]) is at least 1:5.

4. The method according to claim any of the preceding claims, wherein the reagent has the general formula [a]-[linker$_1$]$_n$-[b]-[-linker$_2$]$_m$-[c], wherein n is 0, 1 or 2, m is 0, 1 or 2, [linker$_1$] and [linker$_2$] are independently selected from the group of an optionally substituted hydrocarbyl group and a non-proteolytic hydrocarbyl group, preferably wherein n is 0 or 1, m is 1 and linker$_2$ is a non-proteolytic hydrocarbyl group.

5. The method according to any of the preceding claims, wherein the method comprises the step of contacting the sample with a first reagent and a second reagent, wherein the first reagent has the general formula [a]-[b]-[c] (I) and [b] is a peptide comprising a cleavage site (b') and the second reagent has the formula [a]-[d]-[c] (III), wherein [d] is a peptide comprising a cleavage site (d'), wherein the cleavage site d' is different to the cleavage site b'.

6. The method according to any of the preceding claims, wherein step i) comprises the step of adding a diluent, preferably wherein the diluent comprises a buffering agent selected from the group consisting of MOPS, phosphate, citrate, HEPES TRIS-HCl, phosphate buffered saline, the volume ratio of diluent to sample preferably being in the range of 0.1 to 100, more preferably 0.5 to 75, even more preferably in the range 1 to 50.

7. The method according to any of the preceding claims, wherein the concentration of reagent in the sample receptacle after addition of the sample, and optionally a diluent, is in the range of 0.01 to 10 uM, preferably 0.02 to 8 uM, more preferably 0.05 to 5 uM, even more preferably 0.1 to 2.5 uM.

8. The method according to any of the preceding claims, wherein

- the fluorescent agent having an emission wavelength of 650-900 nm comprises a cyanine moiety, preferably a sulfo-cyanine moiety and wherein the non-fluorescent agent having an absorption wavelength of 650-900 nm comprises a cyanine moiety, preferably a sulfo-cyanine moiety and/or
- an increase in fluorescence emission in the range of 700-850 nm, more preferably in the range of 750-850 nm is indicative of infection.

9. The method according to any of the preceding claims, wherein

- the bacterial biomarker is a bacterial membrane bound transpeptidase or an extracellular bacterial protease, and/or
- the cleavage site is specific for a bacterial biomarker for a bacteria species selected from the group consisting of *Bacillus cereus, Staphylococcus aureus, Streptococcus pyogenes, Pseudomonas aeruginosa, Escherichia coli* and combinations thereof, preferably wherein the cleavage site is specific for *Pseudomonas aeruginosa and Bacillus cereus,* more preferably wherein the cleavage site is specific for *Pseudomonas aeruginosa.*

10. The method according to any of the preceding claims, wherein [b]

- comprises between 3 and 8 amino acids, more preferably between 3 and 7 amino acids, even more preferably between 3 and 6 amino acids,
and/or
- has the general formula $X_{aa1}$,$X_{aa2}$,$X_{aa3}$, wherein:

  $X_{aa1}$ is a small, hydrophobic amino acid, preferably $X_{aa1}$ is selected form the group consisting of alanine, glycine, leucine, valine, norleucine, norvaline, isoleucine, isovaline, alloisoleucine;
  $X_{aa2}$ is preferably a small or aromatic hydrophobic amino acid, preferably, $X_{aa2}$ is selected from the group consisting of alanine, glycine, leucine, valine, norleucine, norvaline, isoleucine, isovaline, alloisoleucine and phenylalanine;

$X_{aa3}$ is selected form the group consisting of alanine, glycine, leucine, valine, norleucine, norvaline, isoleucine, isovaline, alloisoleucine, more preferably $X_{aa3}$ is selected form the group consisting of alanine, glycine, leucine, valine.

**11.** The method according to any of the preceding claims, wherein

- the sample is contacted with the reagent in a container and the fluorescence emitted by the reagent is monitored by a handheld or benchtop fluorescence spectrometer adapted to receive the container,
and/or
- the reagent is not attached to a surface,
and/or
- the sample is not subjected to an ex vivo step of bacterial incubation in the temperature range of 20 to 40°C either prior to or after being contacted with the reagent.

**12.** The method according to any of the preceding claims, wherein

- the bead comprises 5 to 95 wt.% by weight of the bead carbohydrate, preferably 10 to 90 wt.%, more preferably 15 to 85 wt.%, yet more preferably 20 to 80 wt.% carbohydrate, by total weight of the bead,
- and/or
- the carbohydrate is selected from the group consisting of

  - the monosaccharides glucose, fructose, galactose and combinations thereof;
  - the disaccharides sucrose, maltose, lactose, trehalose and combinations thereof; and
  - the polysaccharides alginate, chitosan, hyaluronic acid, cellulose derivatives, dextran and combinations thereof.

**13.** A lyophilized bead as defined in any of the preceding claims, comprising

- a carbohydrate, chosen from the group, consisting of monosaccharide, disaccharide, polysaccharide and combinations thereof, and
- a reagent having the general formula [a]-[b]-[c] (I), wherein:

  [a] is a fluorescent agent having an emission wavelength of 650-900 nm,
  [c] is a non-fluorescent agent having an absorption wavelength of 650-900 nm, for quenching said emission of said fluorescent agent,
  [b] is a peptide comprising a cleavage site, the cleavage site being specific for a bacterial biomarker, wherein the bacterial biomarker is a bacterial membrane bound protease, bacterial membrane bound transpeptidase, intracellular bacterial protease or extracellular bacterial protease.

**14.** A kit for in vitro diagnosing infection in bodily fluid samples, comprising:

a) a container comprising a lyophilized bead of claim 13,
b) a set of instructions for carrying out the method of claims 1-12,
c) optionally a container containing a diluent,

the reagent and the diluent preferably being present in two compartments of a container, preferably wherein the compartments are separated by a pressure activatable membrane.

**15.** A system comprising:

a) a container comprising a lyophilized bead of claim 13,
b) a device adapted to receive the container and monitor the fluorescence signal emitted from the reagent in the presence of a sample of synovial fluid, peritoneal fluid or cerebrospinal fluid with the reagent, wherein the system is adapted to carry out a method according to claims 1-12,

the system preferably being a point-of-care system or a patient beside system.

## Patentansprüche

1. Verfahren zur In-vitro-Diagnose von Infektionen in Körperflüssigkeitsproben unter Verwendung eines Reagenzes der allgemeinen Formel [a]-[b]-[c] (I), wobei:

   [a] ein Fluoreszenzmittel mit einer Emissionswellenlänge von 650 bis 900 nm ist,
   [c] ein nicht fluoreszierendes Mittel mit einer Absorptionswellenlänge von 650 bis 900 nm ist, um die Emission des Fluoreszenzmittels zu löschen,
   [b] ein Peptid ist, das eine Spaltstelle umfasst, wobei die Spaltstelle für einen bakteriellen Biomarker spezifisch ist,

   wobei der bakterielle Biomarker eine bakterielle membrangebundene Protease, eine bakterielle membrangebundene Transpeptidase, eine intrazelluläre bakterielle Protease oder eine extrazelluläre bakterielle Protease ist, wobei das Reagenz in Form eines lyophilisierten Kügelchens bereitgestellt wird, das ein Kohlenhydrat umfasst, das aus der Gruppe ausgewählt ist, die aus Monosaccharid, Disaccharid, Polysacchariden und Kombinationen besteht, wobei das Monosaccharid die allgemeine Formel $(CH_2O)_n$ hat, wobei das Verfahren die folgenden Schritte umfasst:

   i) Inkontaktbringen einer Probe einer Körperflüssigkeit, vorzugsweise einer menschlichen Körperflüssigkeit, mit dem Reagenz in einem Probenbehälter, und
   ii) Überwachen der Fluoreszenzemission im Bereich von 650 bis 900 nm aus der Probe in dem Behälter in Schritt i), wobei eine Zunahme der Fluoreszenzemission im Bereich von 650 bis 900 nm auf das Vorhandensein einer Infektion in der Körperflüssigkeitsprobe hinweist.

2. Verfahren nach Anspruch 1, wobei die Körperflüssigkeitsprobe aus der Gruppe ausgewählt ist, die aus Fruchtwasser, Urin, seröser Flüssigkeit, Synovialflüssigkeit, Peritonealdialyseflüssigkeit, Sperma, Talg und Liquor cerebrospinalis besteht, wobei die Körperflüssigkeit vorzugsweise aus der Gruppe ausgewählt ist, die aus Synovialflüssigkeit, Peritonealdialyseflüssigkeit und Liquor cerebrospinalis ausgewählt ist, wobei die Körperflüssigkeit noch bevorzugter aus der Gruppe ausgewählt ist, die aus Synovialflüssigkeit und Peritonealdialyseflüssigkeit besteht.

3. Verfahren nach Anspruch 1 oder 2, wobei das Massenverhältnis von [b] : ([a]+[c]) mindestens 1:2 beträgt, wobei das Massenverhältnis von [b] : ([a]+[c]) vorzugsweise mindestens 1:3 beträgt, wobei das Massenverhältnis von [b] : ([a] +[c]) noch bevorzugter mindestens 1:4 beträgt, und wobei das Massenverhältnis von [b] : ([a]+[c]) noch bevorzugter mindestens 1:5 beträgt.

4. Verfahren nach einem der vorstehenden Ansprüche, wobei das Reagenz die allgemeine Formel [a]-$[\text{Linker}_1]_n$-[b]-$[\text{Linker}_2]_m$-[c] aufweist, wobei n 0, 1 oder 2 ist, m 0, 1 oder 2 ist, $[\text{Linker}_1]$ und $[\text{Linker}_2]$ unabhängig voneinander aus der Gruppe einer gegebenenfalls substituierten Kohlenwasserstoffgruppe und einer nicht-proteolytischen Kohlenwasserstoffgruppe ausgewählt sind, wobei vorzugsweise n 0 oder 1 ist, m 1 ist und $\text{Linker}_2$ eine nicht-proteolytische Kohlenwasserstoffgruppe ist.

5. Verfahren nach einem der vorstehenden Ansprüche, wobei das Verfahren den Schritt des Inkontaktbringens der Probe mit einem ersten Reagenz und einem zweiten Reagenz umfasst, wobei das erste Reagenz die allgemeine Formel [a]-[b]-[c] (I) hat und [b] ein Peptid ist, das eine Spaltstelle (b') umfasst, und das zweite Reagenz die Formel [a]-[d]- [c] (III) aufweist, wobei [d] ein Peptid ist, das eine Spaltstelle (d') umfasst, wobei sich die Spaltstelle d' von der Spaltstelle b' unterscheidet.

6. Verfahren nach einem der vorstehenden Ansprüche, wobei Schritt i) den Schritt des Hinzufügens eines Verdünnungsmittels umfasst, wobei das Verdünnungsmittel vorzugsweise ein Puffermittel umfasst, das aus der Gruppe ausgewählt ist, die aus MOPS, Phosphat, Citrat, HEPES TRIS-HCl, phosphatgepufferte Kochsalzlösung besteht, wobei das Volumenverhältnis von Verdünnungsmittel zu Probe vorzugsweise im Bereich von 0,1 bis 100, noch bevorzugter 0,5 bis 75, noch bevorzugter im Bereich von 1 bis 50 liegt.

7. Verfahren nach einem der vorstehenden Ansprüche, wobei die Konzentration des Reagenzes im Probenbehälter nach Zugabe der Probe und gegebenenfalls eines Verdünnungsmittels im Bereich von 0,01 bis 10 uM, vorzugsweise 0,02 bis 8 uM, noch bevorzugter 0,05 bis 5 uM, noch mehr bevorzugt 0,1 bis 2,5 uM liegt.

8. Verfahren nach einem der vorstehenden Ansprüche, wobei

- das Fluoreszenzmittel mit einer Emissionswellenlänge von 650 bis 900 nm eine Cyanin-Einheit, vorzugsweise eine Sulfo-Cyanin-Einheit, umfasst und wobei das nicht-fluoreszierende Mittel mit einer Absorptionswellenlänge von 650 bis 900 nm eine Cyanin-Einheit, vorzugsweise eine Sulfo-Cyanin-Einheit, umfasst
und/oder
- eine Zunahme der Fluoreszenzemission im Bereich von 700 bis 850 nm, vorzugsweise im Bereich von 750 bis 850 nm, auf eine Infektion hinweist.

**9.** Verfahren nach einem der vorstehenden Ansprüche, wobei

- der bakterielle Biomarker eine bakterielle membrangebundene Transpeptidase oder eine extrazelluläre bakterielle Protease ist,
und/oder
- die Spaltstelle spezifisch für einen bakteriellen Biomarker für eine Bakterienart ist, die aus der Gruppe ausgewählt ist, die aus *Bacillus cereus, Staphylococcus aureus, Streptococcus pyogenes, Pseudomonas aeruginosa, Escherichia coli* und Kombinationen davon besteht, wobei die Spaltstelle vorzugsweise spezifisch für *Pseudomonas aeruginosa und Bacillus cereus ist,* und die Spaltstelle noch bevorzugter spezifisch für *Pseudomonas aeruginosa ist.*

**10.** Verfahren nach einem der vorstehenden Ansprüche, wobei [b]

- zwischen 3 und 8 Aminosäuren, vorzugsweise zwischen 3 und 7 Aminosäuren, noch bevorzugter zwischen 3 und 6 Aminosäuren umfasst,
und/oder
- die allgemeine Formel $X_{aa1}$,$X_{aa2}$,$X_{aa3}$ aufweist, wobei:

$X_{aa1}$ eine kleine, hydrophobe Aminosäure ist, wobei $X_{aa1}$ vorzugsweise aus der Gruppe bestehend aus Alanin, Glycin, Leucin, Valin, Norleucin, Norvalin, Isoleucin, Isovalin, Alloisoleucin ausgewählt wird;
$X_{aa2}$ vorzugsweise eine kleine oder aromatische hydrophobe Aminosäure ist, wobei $X_{aa2}$ vorzugsweise aus der Gruppe, die aus Alanin, Glycin, Leucin, Valin, Norleucin, Norvalin, Isoleucin, Isovalin, Alloisoleucin und Phenylalanin besteht, ausgewählt wird;
$X_{aa3}$ aus der Gruppe bestehend aus Alanin, Glycin, Leucin, Valin, Norleucin, Norvalin, Isoleucin, Isovalin, Alloisoleucin ist ausgewählt wird, wobei $X_{aa3}$ vorzugsweise aus der Gruppe bestehend aus Alanin, Glycin, Leucin, Valin ausgewählt wird.

**11.** Verfahren nach einem der vorstehenden Ansprüche, wobei

- die Probe in einem Behälter mit dem Reagenz in Kontakt gebracht wird und die vom Reagenz emittierte Fluoreszenz durch ein Hand- oder Tischfluoreszenzspektrometer überwacht wird, das zum Aufnehmen des Behälters ausgelegt ist,
und/oder
- das Reagenz nicht an einer Oberfläche angebracht ist,
und/oder
- die Probe weder vor noch nach dem Kontakt mit dem Reagenz einem Ex-vivo-Schritt der bakteriellen Inkubation im Temperaturbereich von 20 bis 40 °C unterzogen wird.

**12.** Verfahren nach einem der vorstehenden Ansprüche, wobei

- das Kügelchen 5 bis 95 Gew.-% Kohlenhydrat, vorzugsweise 10 bis 90 Gew.-%, noch bevorzugter 15 bis 85 Gew.-%, noch bevorzugter 20 bis 80 Gew.-% Kohlenhydrat, bezogen auf das Gesamtgewicht des Kügelchens, umfasst,
und/oder
- das Kohlenhydrat aus der Gruppe ausgewählt ist, bestehend aus

- den Monosacchariden Glucose, Fructose, Galactose und Kombinationen davon;
- den Disacchariden Saccharose, Maltose, Lactose, Trehalose und Kombinationen davon; und
- den Polysacchariden Alginat, Chitosan, Hyaluronsäure, Cellulosederivaten, Dextran und Kombinationen davon.

**13.** Ein lyophilisiertes Kügelchen gemäß einem der vorstehenden Ansprüche, umfassend

- ein Kohlenhydrat, ausgewählt aus der Gruppe bestehend aus Monosacchariden, Disacchariden, Polysacchariden und Kombinationen davon, und
- ein Reagenz mit der allgemeinen Formel [a]-[b]-[c] (I), wobei

[a] ein Fluoreszenzmittel mit einer Emissionswellenlänge von 650-900 nm ist,
[c] ein nichtfluoreszierender Wirkstoff mit einer Absorptionswellenlänge von 650 bis 900 nm ist, um die Emission des Fluoreszenzmittels zu löschen,
[b] ein Peptid ist, das eine Spaltstelle umfasst, wobei die Spaltstelle für einen bakteriellen Biomarker spezifisch ist, wobei der bakterielle Biomarker eine bakterielle membrangebundene Protease, eine bakterielle membrangebundene Transpeptidase, eine intrazelluläre bakterielle Protease oder eine extrazelluläre bakterielle Protease ist.

**14.** Kit zur In-vitro-Diagnose einer Infektion in Körperflüssigkeitsproben, umfassend:

a) einen Behälter, der ein lyophilisiertes Kügelchen gemäß Anspruch 13 umfasst,
b) eine Anleitung zur Durchführung des Verfahrens gemäß den Ansprüchen 1 bis 12,
c) optional einen Behälter, der ein Verdünnungsmittel enthält,

wobei das Reagenz und das Verdünnungsmittel vorzugsweise in zwei Kammern eines Behälters vorhanden sind, wobei die Kammern vorzugsweise durch eine druckaktivierbare Membran getrennt sind.

**15.** Ein System, umfassend:

a) einen Behälter, der ein lyophilisiertes Kügelchen gemäß Anspruch 13 enthält,
b) eine Vorrichtung, die dazu ausgelegt ist, den Behälter aufzunehmen und das Fluoreszenzsignal zu überwachen, das vom Reagenz in Gegenwart einer Probe von Synovialflüssigkeit, Peritonealflüssigkeit oder Liquor cerebrospinalis mit dem Reagenz emittiert wird, wobei das System dazu ausgelegt ist, ein Verfahren gemäß den Ansprüchen 1-12 durchzuführen,

wobei das System vorzugsweise ein Point-of-Care-System oder ein Patient-Beside-System ist.

## Revendications

**1.** Procédé de diagnostic in vitro d'une infection dans des échantillons de fluides corporels à l'aide d'un réactif répondant à la formule générale [a]-[b]-[c] (I), dans laquelle :

[a] est un agent fluorescent ayant une longueur d'onde d'émission de 650 à 900 nm,
[c] est un agent non fluorescent ayant une longueur d'onde d'absorption de 650 à 900 nm, destiné à éteindre ladite émission dudit agent fluorescent,
[b] est un peptide comprenant un site de clivage, le site de clivage étant spécifique d'un biomarqueur bactérien, dans lequel le biomarqueur bactérien est une protéase liée à la membrane bactérienne, une transpeptidase liée à la membrane bactérienne, une protéase bactérienne intracellulaire ou une protéase bactérienne extracellulaire, le réactif étant fourni sous la forme d'une bille lyophilisée comprenant un glucide, choisi dans le groupe constitué par un monosaccharide, un disaccharide, polysaccharides et leurs combinaisons, le monosaccharide ayant la formule générale $(CH_2O)_n$, dans lequel le procédé comprend les étapes consistant à :

i) mettre en contact un échantillon de fluide corporel, de préférence un fluide corporel humain, avec le réactif dans un récipient à échantillon, et
ii) surveiller l'émission de fluorescence dans la gamme de 650 à 900 nm provenant de l'échantillon dans le récipient à l'étape i), dans lequel une augmentation de l'émission de fluorescence dans la gamme de 650 à 900 nm indique la présence d'une infection dans l'échantillon de fluide corporel.

**2.** Procédé selon la revendication 1, dans lequel l'échantillon de fluide corporel est choisi dans le groupe constitué par le liquide amniotique, l'urine, le liquide séreux, le liquide synovial, le liquide de dialyse péritonéale, le sperme, le sébum et le liquide céphalo-rachidien, de préférence dans lequel le fluide corporel est choisi dans le groupe constitué par le

liquide synovial, liquide de dialyse péritonéale et liquide céphalo-rachidien, de préférence encore dans lequel le fluide corporel est choisi parmi le groupe constitué du liquide synovial et du liquide de dialyse péritonéale.

**3.** Procédé selon la revendication 1 ou 2, dans lequel le rapport massique [b] : ([a]+[c]) est d'au moins 1:2, de préférence le rapport massique [b] : ([a]+[c]) est d'au moins 1:3, de préférence le rapport massique [b] : ([a]+[c]) est d'au moins 1:4, de préférence encore le rapport massique [b] : ([a]+[c]) est d'au moins 1:5.

**4.** Procédé selon l'une quelconque des revendications précédentes, dans lequel le réactif a la formule générale [a]-[linker$_1$]$_n$-[b]-[-linker$_1$]$_m$-[c], dans laquelle n est 0, 1 ou 2, m est 0, 1 ou 2, [linker$_1$] et [linker$_2$] sont choisis indépendamment dans le groupe constitué d'un groupe hydrocarbyle éventuellement substitué et d'un groupe hydrocarbyle non protéolytique, de préférence dans lequel n est 0 ou 1, m est 1 et linker$_2$ est un groupe hydrocarbyle non protéolytique.

**5.** Procédé selon l'une quelconque des revendications précédentes, dans lequel le procédé comprend l'étape consistant à mettre en contact l'échantillon avec un premier réactif et un deuxième réactif, dans lequel le premier réactif a la formule générale [a]-[b]-[c] (I) et [b] est un peptide comprenant un site de clivage (b') et le deuxième réactif a la formule [a]-[d]- [c] (III), dans laquelle [d] est un peptide comprenant un site de clivage (d'), dans laquelle le site de clivage d' est différent du site de clivage b'.

**6.** Procédé selon l'une quelconque des revendications précédentes, dans lequel l'étape i) comprend l'étape consistant à ajouter un diluant, de préférence dans laquelle le diluant comprend un agent tampon choisi dans le groupe constitué par le MOPS, le phosphate, le citrate, HEPES TRIS-HCl, une solution saline tamponnée au phosphate, le rapport volumique entre le diluant et l'échantillon étant de préférence compris entre 0,1 et 100, de préférence entre 0,5 et 75, et de préférence encore entre 1 et 50.

**7.** Procédé selon l'une quelconque des revendications précédentes, dans lequel la concentration du réactif dans le récipient d'échantillon après ajout de l'échantillon, et éventuellement d'un diluant, est comprise entre 0,01 et 10 uM, de préférence entre 0,02 et 8 uM, plus préférablement entre 0,05 et 5 uM, encore plus préférablement entre 0,1 et 2,5 uM.

**8.** Procédé selon l'une quelconque des revendications précédentes, dans lequel

- l'agent fluorescent ayant une longueur d'onde d'émission de 650 à 900 nm comprend un fragment cyanine, de préférence un fragment sulfo-cyanine, et dans lequel l'agent non fluorescent ayant une longueur d'onde d'absorption de 650 à 900 nm comprend un fragment cyanine, de préférence un fragment sulfo-cyanine et/ou
- une augmentation de l'émission de fluorescence dans la gamme de 700 à 850 nm, de préférence dans la gamme de 750 à 850 nm, est indicative d'une infection.

**9.** Procédé selon l'une quelconque des revendications précédentes, dans lequel

- le biomarqueur bactérien est une transpeptidase liée à la membrane bactérienne ou une protéase bactérienne extracellulaire, et/ou
- le site de clivage est spécifique d'un biomarqueur bactérien pour une espèce bactérienne choisie dans le groupe constitué par *Bacillus cereus, Staphylococcus aureus, Streptococcus pyogenes, Pseudomonas aeruginosa, Escherichia coli* et leurs combinaisons, de préférence dans lequel le site de clivage est spécifique de *Pseudomonas aeruginosa et Bacillus cereus,* de préférence dans lequel le site de clivage est spécifique de *Pseudomonas aeruginosa.*

**10.** Procédé selon l'une quelconque des revendications précédentes, dans lequel [b]

- comprend entre 3 et 8 acides aminés, de préférence entre 3 et 7 acides aminés, de préférence encore entre 3 et 6 acides aminés, et/ou
- a la formule générale $X_{aa1}$,$X_{aa2}$,$X_{aa3}$, dans laquelle :

  $X_{aa1}$ est un petit acide aminé hydrophobe, de préférence X $_{aa1}$ est choisi dans le groupe constitué par l'alanine, la glycine, la leucine, la valine, la norleucine, la norvaline, l'isoleucine, l'isovaline, l'alloisoleucine ;

$X_{aa2}$ est de préférence un petit acide aminé hydrophobe ou aromatique, de préférence, $X_{aa2}$ est choisi dans le groupe constitué par l'alanine, la glycine, la leucine, la valine, la norleucine, la norvaline, l'isoleucine, l'isovaline, l'alloisoleucine et la phénylalanine ;
$X_{aa3}$ est choisi dans le groupe constitué par l'alanine, la glycine, la leucine, la valine, la norleucine, la norvaline, l'isoleucine, l'isovaline, l'alloisoleucine, de préférence $X_{aa3}$ est choisi dans le groupe constitué par l'alanine, la glycine, la leucine, la valine.

**11.** Procédé selon l'une quelconque des revendications précédentes, dans lequel

- l'échantillon est mis en contact avec le réactif dans un récipient et la fluorescence émise par le réactif est surveillée par un spectromètre à fluorescence portable ou de paillasse adapté pour recevoir le récipient, et/ou
- le réactif n'est pas fixé à une surface, et/ou
- l'échantillon n'est pas soumis à une étape ex vivo d'incubation bactérienne dans la plage de température de 20 à 40°C avant ou après avoir été mis en contact avec le réactif.

**12.** Procédé selon l'une quelconque des revendications précédentes, dans lequel

- la bille comprend 5 à 95 % en poids de glucides, de préférence 10 à 90 % en poids, de préférence encore 15 à 85 % en poids, et de préférence encore 20 à 80 % en poids de glucides par rapport au poids total de la bille, et/ou
- les glucides sont choisis dans le groupe constitué par
- les monosaccharides glucose, fructose, galactose et leurs combinaisons ;
- les disaccharides saccharose, maltose, lactose, tréhalose et leurs combinaisons ; et
- les polysaccharides alginate, chitosane, acide hyaluronique, dérivés de cellulose, dextrane et leurs combinaisons.

**13.** Bille lyophilisée telle que définie dans l'une quelconque des revendications précédentes, comprenant

- un glucide, choisi dans le groupe constitué par les monosaccharides, les disaccharides, les polysaccharides et leurs combinaisons, et
- un réactif ayant la formule générale [a]-[b]-[c] (I), dans laquelle :

  [a] est un agent fluorescent ayant une longueur d'onde d'émission de 650 à 900 nm,
  [c] est un agent non fluorescent ayant une longueur d'onde d'absorption de 650 à 900 nm, pour éteindre ladite émission dudit agent fluorescent,
  [b] est un peptide comprenant un site de clivage, le site de clivage étant spécifique d'un biomarqueur bactérien, dans lequel le biomarqueur bactérien est une protéase liée à la membrane bactérienne, une transpeptidase liée à la membrane bactérienne, une protéase bactérienne intracellulaire ou une protéase bactérienne extracellulaire.

**14.** Kit pour le diagnostic in vitro d'une infection dans des échantillons de fluides corporels, comprenant :

a) un récipient comprenant une bille lyophilisée selon la revendication 13,
b) un ensemble d'instructions pour mettre en œuvre le procédé selon les revendications 1 à 12,
c) éventuellement un récipient contenant un diluant,

le réactif et le diluant étant de préférence présents dans deux compartiments d'un récipient, de préférence dans lequel les compartiments sont séparés par une membrane activable par pression.

**15.** Système comprenant :

a) un récipient comprenant une bille lyophilisée selon la revendication 13,
b) un dispositif adapté pour recevoir le récipient et surveiller le signal de fluorescence émis par le réactif en présence d'un échantillon de liquide synovial, de liquide péritonéal ou de liquide céphalo-rachidien avec le réactif, dans lequel le système est adapté pour mettre en œuvre un procédé selon les revendications 1 à 12,

le système étant de préférence un système de point de service ou un système au chevet du patient.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016076707 A
- WO 2018224561 A
- WO 2018085895 A
- US 2007275423 A


### Non-patent literature cited in the description


- **DAVID A ARMBRUSTER** ; **TERRY PRY**. *Clin Biochem Rev.*, August 2008, vol. 29 (1), S49-S52